# EUROPEAN PATENT APPLICATION

(11) **EP 0 974 652 A1**
(43) Date of publication of application: **26.01.2000**
(21) Application number: 98911227.1
(22) Date of filing: 07.04.1998
(51) Int. Cl.: C12N 15/12, C12P 21/02, C12N 5/10, C07K 14/47, C07K 16/18, G01N 33/53, G01N 33/574

(54) **CANCEROUS METASTASIS-ASSOCIATED GENE**

(30) Priority: 08.04.1997 JP 10533397
(71) Applicant: BANYU PHARMACEUTICAL CO., LTD., Chuo-ku, Tokyo 103-8416 (JP)
(72) Inventor: MORITA, Masashi Banyu Pharmaceutical Co., Ltd., Ibaraki 300-2611 (JP); ARAKAWA, Hiroharu Banyu Pharmaceutical Co., Ltd., Ibaraki 300-2611 (JP); OHTA, Masataka Banyu Pharmaceutical Co., Ltd., Ibaraki 300-2611 (JP)
(74) Representative: VOSSIUS & PARTNER
(86) International application number: JP9801592
(87) International publication number: WO9845431

(57) **Abstract**

A gene exhibiting stronger expression in mouse IMC-HM cells than in mouse IMC-LM cells and isolated by using the differential display method. When the relation between the expression of the isolated gene and cancerous metastasis is examined, it is found out that a transformant with the larger expression dose of the antisense mRNA to the gene shows the more lowered cancerous metastasis ability. By using the isolated protein, cancerous metastasis inhibitors can be screened.

## Description

### Technical Field

The present invention relates to a protein associated with cancer metastasis and a gene encoding the same.

### Background Art

In spite of the remarkable development of methods of diagnosis and treatment, cancer is still one of the main causes of death in most developed countries, including Japan. In malignant cancer, cancer cells not only randomly proliferate but also invade (infiltrate) surrounding organs and metastasize into distant organs. In fact, the majority of deaths due to cancer are caused by its recurrence by metastasis. Therefore, it seems possible to completely cure cancer by the surgical excision of cancerous foci if the metastasis or invasion of cancer cells is effectively suppressed. Now that a method for treating localized cancer cells is about to be established, the most preferred subject for improving the treatment of cancer is now likely to shift to how to treat metastasis. Hence, analyses of genes associated with the invasion and metastasis of cancer cells and proteins as gene products thereof are extremely important for developing new cancer treatment methods.

Hitherto, a number of cancer metastasis-associated genes have been reported. For example, "nm23" was discovered by the differential hybridization method in mouse melanoma K-1735 subtypes exhibiting different colony forming abilities when they are transplanted through mouse tail vein to the lung. Its metastasis potency and expression in Northern blot are inversely correlated. Transfer of cDNA of "nm23" into a highly metastatic cell clone has been reported to result in the reduction of experimental metastasis potency (cf. Steeg, P. S. et al., J. Natl. Cancer Inst., 80, 200-204 (1988) and Leone,A. et al., 25-35 (1991)). Also, a gene highly expressed in low metastatic cell lines was isolated by the differential hybridization method performed with low and high metastatic cells derived from rat prostate cancer R-3327 cell line; said gene was reported to encode fibronectin. In addition, a 4- to 8-fold reduction of the expression of mRNA of fibronectin was reported in many cell clones which became highly metastatic by allowing said clones to express the v-Ha-ras gene (cf. Schalken, J. A. et al., Cancer Res., 48, 2042-2046 (1988)). Furthermore, a number of genes including KAI1 (Dong, J. T. et al., Science, 268, 884-886 (1995)), stromelysin-3 (Basset, P. et al., Nature, 348, 699-704 (1990)), pGM21 (Phillips, S. M. et al., J. Natl. Cancer Inst., 82, 199-203 (1990)), etc. have been reported as cancer metastasis-associated genes.

However, most of these reports were limited only to studies with the reduction of migration and invasion abilities of cancer cells, in which the expression of a target gene had been suppressed, as indicators. No report has appeared on the suppression effect actually demonstrated in the assay system using experimental animals.

### Disclosure of the Invention

An objective of the present invention is to provide a novel protein associated with cancer metastasis and DNA encoding said protein. Another objective of this invention is to provide a vector carrying said DNA, a transformant harboring said vector, and a method for preparing a recombinant protein that comprises culturing said transformant. Still another objective of this invention is to provide DNA used for detecting, isolating, and amplifying said gene, or for suppressing the expression thereof. A further objective of this invention is to provide a method of screening a cancer metastasis inhibitor using proteins and genes associated with cancer metastasis.

Mouse IMC-HM cells, mutant cells isolated from mouse IMC cell line by Arakawa et al., are highly metastatic to the liver. When IMC-HM cells are subcutaneously transplanted to the ventral of mice, first they spontaneously metastasize to the liver, then rapidly metastasize to the main organs of the whole body, leading to the death of the host in about two weeks. In contrast, almost no metastasis potency is observed with IMC-LM cells, a subculture of the parental IMC cells. Mice transplanted with said cells survive for at least six weeks after the transplantation, and no metastatic foci are observed at all with the naked eye (cf. Arakawa, H. et al., Jap. J. Cancer Res., 87, 518-523 (1996)). Although it may be readily assumed that these two cell lines share a very close genetic background, they widely differ in properties such as the presence or absence of spontaneous metastasis potency to distant organs. In view of these facts, the present inventors suspected the presence of a key gene controlling cancer metastasis in mouse IMC-HM cells, and attempted to isolate a strongly expressed gene from mouse IMC-HM cells as compared with mouse IMC-LM cells using the differential display method. As a result, the present inventors succeeded in isolating two mouse cDNA fragments, which are candidates for the factor inducing cancer metastasis. Furthermore, they screened a cDNA library derived from mouse IMC-HA1 Cells using these cDNA fragments as probes, and succeeded in isolating full-length cDNA thereof

The present inventors also investigated the relation between the expression of isolated mouse cDNAs and cancer metastasis. Specifically, they constructed a vector expressing antisense-mRNA against one of the isolated mouse cDNAs (mouse CMAP), and expressed said vector in IMC-HA1 cells derived from IMC-HM cells to prepare many transformants which exhibited different expression levels of the antisense mRNA. The inventors then transplanted the transformants into mice to investigate their cancer-metastasis abilities. As a result, it was found that the higher the antisense MRNA expression level of the transformant is, the lower its cancer metastasis potency is. The inventors also succeeded in isolating human cDNA (human CMAP) that exhibited a high homology with mouse CMAP by the polymerase chain reaction using primers prepared based on the nucleotide sequence of isolated mouse CMAP.

Since a close relation was recognized between the expression of isolated genes and cancer metastasis, the present inventors found it was possible to screen cancer metastasis inhibitors using these proteins and genes.

The present invention relates to a novel protein and gene associated with cancer metastasis, and to a method of using them to screen cancer metastasis inhibitors. More specifically, the present invention relates to:
(1) a protein comprising any one of the amino acid sequences set forth in SEQ ID NOs: 4, 6, 9, or 38, or a protein comprising any one of said amino acid sequences having substitution, deletion, or addition of one or more amino acids and having cancer metastasis potency;
(2) a protein encoded by DNA which hybridizes with DNA comprising any one of the nucleotide sequences set forth in SEQ ID NOs: 3, 5, 7, 8, or 37, said protein having cancer metastasis potency;
(3) a DNA encoding the protein according to (1);
(4) the DNA according to (3) comprising any one of the nucleotide sequences set forth in SEQ ID NOs: 3, 5, 7, 8, or 37;
(5) a DNA hybridizing with a DNA comprising any one of the nucleotide sequences set forth in SEQ ID NOs: 3, 5, 7, 8, or 37, encoding a protein having cancer metastasis potency;
(6) a vector carrying the DNA according to any one of (3) to (5);
(7) a transformant harboring the vector according to (6);
(8) a method for preparing the protein according to (1) or (2), comprising culturing the transformant set forth in (7);
(9) a DNA specifically hybridizing with the DNA according to any one of (3) to (5), comprising at least 15 nucleotide residues;
(10) an antisense DNA against the DNA according to any one of (3) to (5) or a portion thereof;
(11) an antibody binding to the protein according to (1) or (2);
(12) a method for screening a compound having cancer metastasis inhibitory ability, comprising steps of:
   (a) contacting a test sample with the protein according to (1) or (2), and
   (b) selecting compounds having the activity to bind to the protein according to (1) or (2);,
(13) a method for screening compounds having cancer metastasis inhibitory ability, comprising steps of:
   (a) contacting test samples with cells expressing the protein according to (1) or (2),
   (b) detecting the expression level of the protein according to (1) or (2) in cells contacted with test samples using the antibody set forth (11), and
   (c) selecting a compound which reduces the expression level of the protein according to (1) or (2) as compared with that in cells not contacted with test samples.

The present invention relates to a novel protein associated with cancer metastasis. The nucleotide sequence of cDNA isolated and designated "CMAP" by the present inventors is shown in SEQ ID NO: 3, and the amino acid sequence encoded by said cDNA is shown in SEQ ID NO: 4. Mouse "CMAP" cDNA has been isolated by the differential display method as a gene which is highly expressed in a mouse cancer metastatic IMC-HM cell line derived from mouse IMC cells. Its expression was not detected in the IMC-LM cell line, which had been similarly derived from mouse IMC cell line but had almost no detectable cancer metastasis potency (Cf. Example 1). Studies on the expression of mouse CMAP in various cancer cells revealed that it was expressed in M-5076 cells, L5178Y cells, P388 cells, and L1210 cells, but not in IMC-HA1 cells, which formed metastasis foci mainly in the liver when transplanted through the tail vain of mice. In contrast, B-16-BL6 cells and Colon 26 cells, in which the expression of mouse CMAP was not detectable, strongly induced the experimental metastatic foci in the lung, but did not exhibit hepatic metastasis. All other cell lines in which mouse CMAP was expressed below the detection limit were almost completely rejected and regressed when they were transplanted to the tail vein (cf. Example 7). Many transformants that expressed antisense mRNA against a transcript of mouse CMAP cDNA in various levels were prepared and their cancer metastasis abilities *in vivo* were examined by transplanting them into mice. As a result, it was found out that higher expression levels of antisense mRNA of transformant were related with lower cancer metastasis potencies of said transformant (cf. Example 9). These facts indicate a close association of mouse CMAP expression with cancer metastasis.

In addition, the present inventors isolated cDNA (human CMAP cDNA) exhibiting a high homology with mouse CMAP from human spleen by polymerase chain reaction using primers prepared based on the nucleotide sequence of mouse CMAP cDNA. This cDNA's nucleotide sequence is shown in SEQ ID NO: 37, and the amino acid sequence of the protein encoded by said cDNA is shown in SEQ ID NO: 38. Human CMAP had a homology as high as 71.6% with mouse CMAP in the amino acid sequence (Cf. Example 8). This fact strongly indicates that human CMAP functions similarly to mouse CMAP.

Furthermore, as in the case of mouse CMAP, the inventors isolated three cDNA clones (#7.16411, #8.323, and #11.24413) that were overexpressed in mouse IMC-HM cells, by the differential display method using mouse IMC-HM cells and IMC-LM cells. Nucleotide sequences of three cDNA clones are shown in SEQ ID NOs: 5, 7, and 8. Comparison of these nucleotide sequences strongly indicated that "#7.16411" and "#11.24413" are produced when the #8.323 is processed differently. Clones "#7.16411" and "#11.24413" have an identical nucleotide sequence except for about 100 bp at the 5'-end, and were presumed to encode proteins of 131 and 126 amino acids with 20 to 30 different amino acids of the N-terminus. Amino acid sequences of proteins encoded by "#7.16411" cDNA and "#11.24413" cDNA are shown in SEQ ID NOs: 6 and 9, respectively. When the expression patterns of "#7.16411" and "#11.24413" in various cancerous and normal cells were examined, "#7.16411" was detected in almost all cancerous cells and normal tissues in spite of the difference in their expression level. However, "#11.24413" was detected only in cancer cells such as IMC-HA1 cells derived from mouse IMC-HM cells, and P388 cells, and in normal tissues such as the thymus, lung, and spleen and slightly in the peripheral blood (cf. Example 6). These results indicate that #8.323 to "#7.16411" are processed in the majority of normal tissues, and "#11.24413" is assumed to be expressed in specific cells. "#11.24413" may be a factor in providing IMC-HA1 cells with properties such as a rapid invasion of the whole body. The above-described characteristics indicate the possibility of using these proteins for screening compounds having cancer metastasis inhibitory ability.

The protein of this invention may be prepared as a recombinant protein as well as a natural protein using the genetic recombination technique by methods known to those skilled in the art. The natural protein, for example, can be prepared by subjecting extracts of cells or tissues that are expected to express the protein of the present invention to affinity chromatography using the antibody of the present invention described below. The recombinant protein may be prepared by culturing cells transformed with the DNA encoding the protein of this invention as described below.

The present invention also relates to a protein functionally equivalent to the above-described protein (mouse CMAP, human CMAP, #7.16411, and #11.24413). For example, a functionally equivalent protein can be isolated by a method of inducing the amino acid mutation in a protein. Such a method is known to those skilled in the art. Skilled persons would be able to prepare a mutant protein functionally equivalent to the natural protein by appropriately substituting amino acid(s) in the above-described protein (comprising any one of amino acid sequences set forth in SEQ ID NOs: 4, 6, 9, and 38) using Kunkel's method (Kunkel, T. A. et al., Methods Enzymol. 154, 367-382 (1987)), the double primer method (Zoller, H. J. and Smith, H., Methods Enzymol. 154, 329-350 (1987)), the cassette mutation method (Wells, et al., Gene 34, 315-23 (1985)), the megaprimer method (Sarkar, G. and Sommer, S. S., Biotechniques 8, 404-407 (1990)), etc. Amino acid mutation of a protein may also occur spontaneously. Such a mutant protein derived from a natural protein by substituting, deleting, or adding amino acids in its amino acid sequence, and proteins functionally equivalent to the natural protein are also included in the protein of the present invention. Here, "functionally equivalent" means that the protein has cancer metastasis potency. Cancer metastasis potency of the protein can be detected by, for example, suppressing the expression or function of said protein in metastatic cancer cells (for example, using the antisense technique), transplanting said suppressed cells into an animal body, and examining its cancer metastasis potency (Cf. Example 9). The extent of amino acid mutation in the functionally equivalent protein compared with the natural type protein is usually 10% or less of the total amino acids, preferably 10 amino acids or less, more preferably 3 amino acids or less, still preferably 1 amino acid.

Another method known in the art for isolating the functionally equivalent protein is the hybridization technique (Hanahan, D. and Meselson, H., Method. Enzymol. 100, 333-342 (1983) and Benton, W. D. and Davis, R. W., Science 196, 180-182 (1977)). Those skilled in the art can easily obtain a protein functionally equivalent to the above-described protein from DNA that is isolated based on the DNA sequences (SEQ ID NOs: 3, 5, 7, 8, and 37) encoding the above-described protein or a portion thereof and highly homologous with said DNA sequences. Such a protein encoded by DNA hybridizing with the DNAs encoding the above-described proteins and functionally equivalent to said proteins is also included in the protein of the present invention. Here, "functionally equivalent" means that the protein has cancer metastasis potency similarly as described above. Hybridization for isolating functionally equivalent proteins is usually performed in 6 x SSC and 40% formamide at 25°C and subsequent washing with 1 x SSC at 55°C. Preferably, hybridization is performed in 6 x SSC and 40% formamide at 37°C and washing with 0.2 x SSC at 55°C. More preferably, hybridization is performed in 6 x SSC and 50% formamide at 37°C and washing with 0.1. x SSC at 62°C. Those skilled in the art can obviously realize such stringent hybridization conditions by appropriately selecting the dilution rate of SSC, concentration of formamide, temperature, etc. Animals from which functionally equivalent proteins are isolated include human, mouse, rat, rabbit, sheep, cow, dog, and pig, but are not limited thereto. A DNA encoding the protein thus isolated usually has high homology with a nucleotide sequence of cDNA encoding the above-described protein (nucleotide sequences set forth in SEQ ID NOs: 3, 5, 7, 8, and 37). High homology means a sequence identity of at least 70% or more, preferably 80% or more, and more preferably 95% or more, at the nucleotide level.

In addition, the present invention relates to DNA encoding the above-described protein of the present invention (mouse CMAP, human CMAP, #7.16411, #11.24413, and a protein functionally equivalent thereto). There are no limitations in the type of the DNA of this invention as long as it can encode the protein of the invention. The DNA of this invention thus includes synthetic DNA as well as cDNA and genoinic DNA. The DNA of this invention can be isolated by methods known in the art. For example, cDNA can be screened by labeling cDNA encoding the protein of this invention (for example, cDNA comprising any one of nucleotide sequences set forth in SEQ ID NOs: 3, 5, 7, 8, and 37) or a fragment thereof, RNA complementary to said cDNA, or a synthetic oligonucleotide comprising a portion of the nucleotide sequence of said cDNA with ³²P, etc., and then hybridizing the labeled product with a cDNA library derived from cells or tissues expressing the protein of this invention. Alternatively, said cDNA can be cloned by synthesizing oligonucleotides corresponding to the nucleotide sequence of said cDNA, and amplifying them using a polymerase chain reaction with cDNA derived from appropriate cells or tissues as a template. Genomic DNA can be screened by hybridizing a genomic DNA library with a probe such as cDNA encoding the protein of the present invention (for example, cDNA comprising the nucleotide sequence of SEQ ID NO: 3, 5, 7, 8, or 37), a portion thereof, complementary RNA, or a synthetic oligonucleotide containing a part of the sequence of said DNA, which are labeled with ³²P and the like. Alternatively, genomic DNA can be cloned by amplification using polymerase chain reaction with synthetic oligonucleotide primers having a sequence corresponding to the nucleotide sequence of the cDNA. Synthetic DNA can be prepared by, for example, chemically synthesizing oligonucleotides comprising a partial sequence of cDNA encoding the protein of the present invention (for example, cDNA comprising the nucleotide sequence set forth in SEQ ID NOs: 3, 5, 7, 8, or 37), annealing them to form a double strand, and ligating them using a DNA ligase (Khorana, H. G. et al., J. Biol. Chem. 251, 565-570 (1976); and Goeddel, D. V. et al., Proc. Natl. Acad. Sci. USA 76, 106-10 (1979)).

The DNA thus prepared is useful for producing a recombinant protein. The protein of the present invention can be prepared as a recombinant protein by inserting DNA encoding the above-described protein of this invention (for example, cDNA comprising the nucleotide sequence set forth in SEQ ID NOs: 3, 5, 7, 8, or 37) into an appropriate expression vector, introducing said vector into appropriate cells, culturing transformants thus obtained, and purifying a protein thus expressed. Specifically, when *Escherichia coli* is a host, a plasmid vector such as pET-3 (Rosenberg, A. H. et al., Gene 56, 125-35 (1987)), pGEX-1 (Smith, D. B. and Johnson, K. S., Gene 67, 31-40 1988)), etc. can be used. *Escherichia coli* can be transformed by Hanahan's method (Hanahan, D., J. Mol. Biol. 166, 557-580 (1983)), the electroporation method (Dower, W. J. et al., Nucl. Acids Res. 16, 6127-6145 (1988)), etc. A recombinant protein can be synthesized in the form of a fused protein in which it binds to the tag of histidine residues, glutathione S-transferase (GST), etc. at the N-terminus, etc., and can be purified by binding said fused protein to metal-chelate resin, GST-affinity resin (Smith, M. C. et al., J. Biol. Chem. 263, 7211-7215 (1988)), etc. The desired protein can be developed from the fused protein by cleaving the fused protein with thrombin blood coagulating factor Xa, etc. When a fission yeast, *Schizosaccharomyces pombe*, is a host, pESP-1 (Lu, Q. et al., Gene 200, 135-144 (1977)) or the like plasmid vector is used. For example, yeast is transformed by the spheroplast method (Beach, D. and Nurse, P., Nature 290, 140 (1981)), lithium acetate method (Okazaki, K. et al., Nucl. Acids Res. 18, 6485-6489 (1990)), etc. When pESP-1 is used, a recombinant protein is synthesized as a fusion protein with glutathione S-transferase (GST) and is purified by binding the fusion protein to GST-affinity resin. The protein of interest can be separated from the fused protein by cleaving it with thrombin, blood coagulating factor Xa, etc. When mammalian cells such as CHO cells derived from Chinese hamster ovary, human HeLa cells, etc. are the host, pMSG (CLONTECH) or the like vector is used. The recombinant DNA can be introduced into mammalian cells by the calcium phosphate method (Graham, F. L. and van derEb, A. J., Virology 52, 456-467 (1973)), DEAE-dextran method (Sussman, D. J. and Milman, G., Mol. Cell. Biol. 4, 1641-1643 (1984)), lipofection method (Felgner, P. L. et al., Proc. Natl. Acad. Sci. USA 84, 7413-7417 (1987)), electroporation method (Neumann, E. et al., EMBO J. 1, 841-845 (1982)), etc. When insect cells are the host, the baculovirus vector pBacPAK8/9 (Clontech) or similar vectors are used. For example, insect cells can be transformed according to methods as described in Bio/Technology, 6, 47-55 (1980).

The present invention also relates to DNA specifically hybridizing with the above-described DNA of the present invention and having at least 15 nucleotide residues. Here, "specifically hybridize" means that DNA does not cross-hybridize with other DNAs encoding other proteins under stringent conditions. Such DNA can be utilized as a probe for detecting and isolating DNAs encoding the protein of this invention, and as a primer for amplifying said DNAs.

The present invention also relates to antisense DNA against the DNA encoding the protein of the present invention or a portion thereof. Such antisense DNA is utilized to suppress the expression of the protein of the present invention. For example, it is possible to suppress the cancer metastasis ability of said cells by expressing such antisense DNA in cells with cancer metastasis potency to suppress the expression of the protein of this invention. In order to display the antisense effect, the antisense DNA is at least 15 bp or more, preferably 100 bp or more, and usually shorter than 800 bp, preferably shorter than 600 bp.

Furthermore, this invention relates to an antibody binding to the protein of the present invention. Antibodies binding to the protein of the present invention can be prepared by methods known in the art (cf. "Shin Seikagaku Jikkenkoza 1, Proteins I, 389-406, Tokyo Kagaku Dojin"). Polyclonal antibodies can be prepared as follows. A suitable amount of the above-described protein or peptide is administered to animals to be immunized such as rabbits, guinea pigs, mice, or chickens. The antigen may be administered together with an adjuvant (such as FIS and FCA) to stimulate the antibody production. Immunization is usually performed every several weeks. Repeated immunization allows elevating the antibody titer. After the final immunization, antiserum can be obtained by collecting the blood from immunized animals. Polyclonal antibodies can be prepared by fractionating the antiserum using the ammonium sulfate precipitation, anion exchange chromatography and affinity chromatography with protein A or the immobilized antigen. Monoclonal antibodies can be prepared as follows. The protein of this invention or a partial peptide thereof is administered to animals to be immunized as described above. After the final immunization, the spleen or lymph node is excised from immunized animals. Antibody-producing cells contained in the spleen or lymph node are fused with myeloma cells using polyethylene glycol to prepare hybridomas. The hybridomas of interest are then screened and cultured. Monoclonal antibodies can be prepared from the culture supernatant and purified by fractionation using the ammonium sulfate precipitation, anion exchange chromatography and affinity chromatography with protein A or the immobilized antigen. Antibodies thus prepared can be used not only for affinity purification of the protein of this invention but also for diagnosis and antibody treatment of diseases (e.g. cancer) caused by the abnormal expression of the protein of this invention, and detection of the expression level of the protein. For antibody treatment, the antibody to be used is preferably a humanized antibody or human antibody. Humanized antibody, for example, a mouse-human chimeric antibody, can be prepared by isolating the antibody gene from mouse cells producing the antibody against the protein of this invention, recombining the gene of the constant region of H chain with that of the constant region of human IgE H chain, and introducing the resulting recombinant gene into mouse myeloma J558L cells (Neuberger, N. S. et al., Nature 314, 268-270 (1985)). Human antibodies can be prepared by immunizing mice, whose immune system has been replaced with that of humans, with the protein of this invention.

The present invention also relates to a method for screening a compound with cancer metastasis suppressing potency using the protein of this invention. One embodiment of the screening method according to this invention comprises steps of (a) contacting the protein of this invention with test samples, and (b) selecting a compound that binds to the protein of this invention. Test samples used for the screening include purified proteins, expression products of genes (including gene library), extracts of tissues or cells, supernatant of cell cultures, synthetic low molecular weight compounds, and metabolites of microorganisms, but are not limited to them. Test samples used for the screening may be appropriately labeled with a radioactive substance, a fluorescent substance, etc., but are not limited to them. Binding of test samples with the protein of this invention can be detected by the label on a compound bound to the protein of this invention (for example, detecting the binding by the radioactivity or fluorescence intensity). When the TWO-hybrid system is used (Zervos et al., Cell 72, 223-232 (1994) and Fritz et al., Nature 376, 530-533 (1995)), the binding can be detected by examining the activity of the reporter gene. Besides the above-described TWO-hybrid system, various methods known to those skilled in the art can be applied to screening compounds binding to the protein of this invention. The methods include the affinity purification method using a column to which the protein of this invention is immobilized and various peptide display methods such as the phage display method (F. Parmly and G. P. Smith, Gene, 73, 305 (1988)).

Another embodiment of the screening method of this invention comprises steps of (a) contacting test samples with cells expressing the protein of this invention, (b) detecting the expression level of the protein of this invention in the cells contacted with said test samples using an antibody binding to the protein of this invention, and (c) selecting a compound that reduces the expression level of the protein of this invention as compared with that in cells not contacted with said test samples. In this screening method, there are no limitations in the type of cells to be treated with test samples, as long as they express the protein of this invention. Cells to be treated may thus include IMC-HM cells or cells derived from them such as M-5076 cells and L5178Y cells. Test samples may include purified proteins, expression products of genes (including libraries), extracts of tissues or cells, supernatants of cell cultures, synthetic compounds of low molecular weights, and metabolites of microorganisms but are not limited to these. Many known methods, including the ELISA method, immune precipitation method, and Western blotting method, are available without limitation for detecting the expression level of the protein of this invention using an antibody. The antibodies used are labeled for detecting the protein of this invention if required. Examples of labels include enzymes, radioactive substances, and fluorescent substances, but are not so limited. It is also possible to detect the protein of this invention without labeling the antibody of the present invention but by labeling the molecule specifically binding to the antibody of this invention such as a secondary antibody or protein A. A desired compound can also be screened by detecting the expression of mRNA as well as that of the protein described above. The expression of mRNA can be detected by, for example, reverse transcription PCR (RT-PCR ). G3PDH can be used as a control. Compounds isolated by the above screening methods may be candidates for suppressing the activity of the protein of this invention. These compounds can be utilized as cancer metastasis inhibitors.

Effects of the compounds isolated by the above-described screening methods are preferably confirmed *in vivo* as occasion demands. One embodiment of the *in vivo* detection method comprises steps of (a) contacting a compound isolated by the above-described screening method with cells with cancer metastasis potency, (b) transplanting said cells to a nonhuman test mammal, and (c) detecting the cancer metastasis in said nonhuman test mammal to determine whether said compound suppresses cancer metastasis. When mice are used as the test animal, mouse IMC-HM cells or those derived from them are preferably used as the cells with cancer metastasis ability. In this case, cells can be transplanted to mice subcutaneously or through the tail vein. Cancer metastasis can be detected by examining the formation of metastasis foci, for example, in the liver.

Another embodiment of the *in vivo* detection of cancer metastasis suppressing ability of the compound is the method of directly administering the test compound to mice. This method comprises steps of (a) administering a compound isolated by the above-described screening method to nonhuman test mammals, (b) transplanting cells with cancer metastasis potency to said nonhuman test mammals, and (c) detecting cancer metastasis in said nonhuman test mammals to determine whether said compound suppresses cancer metastasis. The compound can be administered orally, intraperitoneally, through the tail vein, subcutaneously, intracutaneously, or intramuscularly. As in the above-described method, when mice are used as the test animal, cells with cancer metastasis ability are preferably mouse IMC-HM cells or those derived therefrom. These cells can be transplanted to mice subcutaneously or through the tail vein, etc. Cancer metastasis can be detected by examining the formation of metastasis foci, for example, in the liver.

### Brief Description of the Drawings

Figure 1 is a photograph showing electrophoretic patterns of CMAP expressed in various cancer cells detected by Northern blotting.
Figure 2 is a photograph showing electrophoretic patterns of CMAP expressed in normal tissues detected by Northern blotting.
Figure 3 compares amino acid sequences between CMAP and human family-2 cystatin.

### Best Mode for Implementing the Invention

The present invention will be described in more detail below with reference to examples, but is not to be construed to be limited to these examples.

### Example 1 Cloning of cancer metastasis-associated gene

### (1) Extraction of the total RNAs from IMC-HM and IMC-LM cells

The total RNAs were extracted from IMC-HM and IMC-LM cells using ISOGEN (Nippon Gene). First, cultured cells in the logarithmic growth phase (1x 10⁷ cells) were collected and sedimented by centrifugation. After the culture supernatant was removed by aspiration, ISOGEN (1 ml) was added to the cells, and the mixture was allowed to stand at room temperature for 5 min to lyse the cells. Chloroform (0.2 ml) was then added to the mixture, and the resulting mixture was vigorously vortexed for 15 sec, allowed to stand at room temperature for several minutes, and centrifuged (4°C) at 12,000 x g for 15 min. The aqueous layer containing RNA was collected, and an equal volume of isopropanol was added thereto. The mixture was stirred, allowed to stand at room temperature for 10 min, and centrifuged again under cooling at 12,000 x g for 15 min. RNA thus sedimented was collected, rinsed with ethanol, and dissolved in sterilized pure water (50 µl), which had been treated with diethyl pyrocarbonate (DEPC) to obtain a total RNA solution. When cultured cells (5 x 10⁴ cells) from which the total RNA was isolated were transplanted subcutaneously into the ventral of a five-week old female CDF1 mouse, observation with the naked eye revealed that IMC-HM cells metastasized to the liver of the host mouse to kill it in two weeks, while IMC-LM cells did not metastasize to the liver, allowing it to live for six weeks.

### (2) Preparation of single-stranded cDNA using reverse transcriptase

A complementary single-stranded DNA was prepared with the total RNA obtained above as a template using reverse transcriptase Superscript II (GIBCO BRL) derived from MMLV and an mRNA Fingerprinting Kit (Clontech). A cDNA synthesis primer (oligo dT primer) (1 µl) was added to the total RNA (2 µg), and the total mixture volume was made 5 µl by adding DEPC-treated, sterilized pure water. The mixture was kept at 70°C for 3 min then quickly cooled. A mixed solution (total volume 5 µl) of the reaction buffer (5 x first strand buffer) (2 µl), dNTP mix (each 5 mM) (2 µl), and Superscript II (200 unit/µl) (1 µl) was added to this mixture, and the resulting mixture was allowed to stand at 42°C for 1 hour in an air incubator. After the reaction was completed, the mixture was treated at 72°C for 10 min, diluted 160-fold with the sterilized pure water, and stored at -20 °C . The RNA fingerprinting was then performed based on polymerase chain reaction (PCR) using this single-stranded cDNA solution.

### (3) Amplification of single-stranded cDNA by PCR

Equal amounts of LA Tag DNA polymerase (Takara Shuzo) (5 U/ µl) and undiluted solution of TaqStart Antibody (registered trade mark, Clontech) were mixed, and the mixture was allowed to stand at room temperature for a few minutes to make a 50 x polymerase mix. This mix (0.4 µl) was combined with 10 x LA PCR buffer (Takara Shuzo) (2 µl), sterilized pure water (13.9 µl), dNTP mix (Clontech) (5 mM each) (0.2 µl), and [³⁵S]dATPαS (Amersham) (0.5 µl), and the resulting mixture was used as a PCR master mix. To the stock solution of the single-stranded cDNA (1 µl) were added 20 µM of P primer (P1-10/SEQ ID NOs: 10 to 19), 20 µM of T primer (T1-9/SEQ ID NOs: 20 to 28) (Clontech) (1 µl each), and the above-described PCR master mix (17 µ1). The resulting mixture was overlaid with mineral oil (Sigma), and PCR was performed in a DNA thermal cycler PJ2000 (PERKIN ELMER CETUS) to amplify several genes at once. The PCR comprises one cycle of 94°C for 5 min, 40°C for 5 min, and 68°C for 5 min; two cycles of 94°C for 2 min, 40°C for 5 min, and 68°C for 5 min; 25 cycles of 94°C for 1 min, 60°C for 1 min, and 68°C for 2 min; and finally 68°C for 7 min.

### (4) Separation of PCR products by electrophoresis

A plate for the sequence gel (20 x 40 cm) was assembled using a spacer according to the standard method. A 6% glycerol-resistant gel and glycerol-resistant buffer (United States Biochemical) were used. PCR products (3.5 µl each) amplified with P primer and T primer were mixed with a reaction termination solution (2 µl each), heated at 94°C for 2 to 3 min, and then cooled. The resulting mixture (3 µ 1 each) was loaded onto the glycerol-resistant gel and electrophoresed at 35 W for about 3 hours. After electrophoresis, the gel was transferred onto a filter paper, which was dried at 80°C for 2 hours in a gel dryer. Radioactivity in the gel was copied on an imaging plate for BAS2000 (Fuji Film) by leaving it on said plate overnight, and imaged with BAS2000.

This imaging analysis was used to search for genes whose expression level significantly differred between IMC-HM cells and IMC-LM cells. If the expression level of one gene is predominantly high in IMC-HM cells, said gene can be a candidate of a gene that induces cancer metastasis. Furthermore, if the expression level of one gene is predominantly high in IMC-LM cells, said gene would be a candidate of a gene that suppresses cancer metastasis. Two bands of possible candidates for the factor inducing metastasis were found from IMC-HM cells. One band was detected in PCR products obtained from a primer set of P2 and T3, and the other from a primer set of P2 and T8. The former band was designated "23-1," and the latter band, "28-1."

### (5) Cloning of IMC-HM cell-specific cDNA bands

IMC-HM cell-specific cDNA bands 23-1 and 28-1 were excised directly from the dried gel, and each gel segment was boiled in sterilized pure water (40 µl) for 10 min to elute the desired cDNA fragments. This minute quantity of cDNA fragment was re-amplified as follows.

10 x EX Tag buffer (Takara Shuzo) (5 µl), dNTP mix (each 2.5 mM) (1 µl), EX Taq DNA polymerase (Takara Shuzo) (5 U/µl) (0.5 µl), and sterilized pure water (31.5 µl) were mixed to make a PCR master mix. The eluted cDNA fragment (7 µl) and the P primer and T primer (2.5 µl each) which had been used for detecting said cDNA band were added to this mix. The mixture was overlaid with mineral oil and subjected to PCR comprising 20 cycles of 94°C for 1 min, 60°C for 1 min, and 68°C for 2 min in a DNA thermal cycler PJ2000 (PERKIN ELMER CETUS) to specifically amplify the desired cDNA fragment. PCR products were electrophoresed on 1% agarose gel using a Mupid (COSMOBIO) at 50 V for 1 hour and identified by staining with ethidium bromide.

In general, PCR products of cDNA have additional A at their 3' -terminus, and may be directly cloned by a TA type vector (having T at its terminus). Therefore, cDNA fragments thus re-amplified were cloned using a TA cloning kit (Invitrogen) according to the following method. A 10 x ligation buffer (Invitrogen) (1 µl), pCR II vector (registered trade mark, Invitrogen) (25 ng/ml) (2 µl), sterilized pure water (9 µl), and T4 DNA ligase (Invitrogen) (1 µl) were added to re-amplified PCR fragments (5 µl), and the mixture was allowed to stand at 14°C overnight to insert the cDNA. A tube of One Shot INVαF' competent cells (registered trade mark, Invitrogen) was mixed with 0.5 M β-mercaptoethanol (50 µl). The above-described vector previously ligated with cDNA (2 µl) was added to this mixture, and the resulting mixture was incubated at 42°C for 30 sec. After standing on ice for 2 min, an SOC medium (450 µl) was added, and the mixture was shake-cultured at 37°C for 1 hour. The solution obtained after the above transformation procedure (100 µl and 200 µl) was spread onto a previously prepared LB agar plate containing 50 µg/ml kanamycin and X-Gal and allowed to stand at 37°C overnight. The vector without the insertion reacts with X-Gal to turn blue while the one with cDNA incorporated turns white. According to this principle, 10 white colonies were picked up and incubated again to be used for sequencing; at the same time, replicas were prepared. Plasmids were prepared from each transformant cell strain using a QIAprep Spin Plasmid Kit (QIAGEN) by the following procedure.

Each transformant cell strain was incubated in an LB medium (3 ml) containing 50 µg/ml kanamycin at 37°C for about 8 hours and centrifuged to collect the cells. These cells were dissolved in buffer P1 (QIAGEN) (250 µl), mixed with buffer P2 (QIAGEN) (250 µl), further mixed with buffer P3 (QIAGEN) (350 µl), and then centrifuged at 10,000 x g for 10 min. The supernatant was transferred onto a QIAprep Spin column (QIAGEN) and centrifuged at 10,000 x g for 1 min. The column was washed with buffer PB (QIAGEN) to remove the nuclease activity and further washed with the buffer PE (QIAGEN) (0.75 ml). The plasmid retained in the column was then eluted with the TE buffer and collected. The concentration of the plasmid was calculated from the A260 value determined with a Hitachi spectrophotometer (U-3300).

### (6) Sequencing of the cloned IMC-HM cell-specific cDNA fragment

A nucleotide sequence of cDNA was determined by the dye terminator method (equipment used, ABI Model 377). Although performance conditions may vary depending on the purification grade and concentration of plasmid serving as a template, in general the sequence consisting of 500 to 600 nucleotides may be determined at the same time using the dye terminator method. pCR II vector has the forward and reverse primer sites of M13 near the insertion site. Using each primer previously fluorescence-labeled, both strands were compared to determine the respective sequences. AmpliTaq (25 µl), 5 x buffer (25 µl), and purified water (125 µl) were mixed to dilute the enzyme, and premix was prepared for each of A, C, G, and T. To prepare premix of A or C, equal volumes of d/ddNTP, dye-primer, 5 x buffer, and the diluted enzyme (25 µl each) were mixed; the premix of G or T was prepared by mixing 50 µl each of these ingredients. The template DNA (100 to 200 ng/µl) (1 µl) and the premixes of A and C (4 µl each), and said template DNA (2 µl) and the premixes of G and T (8 µl each) were separately mixed and subjected to cycle sequencing reaction with 25 cycles of 96°C for 2 min, 96°C for 10 sec, 50°C for 5 sec, and 60°C for 4 min using a PERKIN ELMER 9600. Four clones randomly selected from 10 each cloned plasmids were sequenced, and all of them were found to have identical nucleotide sequences. One of the plasmid clones obtained from 23-1 was then designated 23-1#2, and that obtained from 28-1 was designated 28-1#3.

Next, sequences determined for 23-1#2 (SEQ ID NO: 1) and 28-1#3 (SEQ ID NO: 2) were analyzed with a computer using software from Genetics Computer Group, Wisconsin Package version 8.1. GenBank and EMBL database searches for the sequence homology with a FastA revealed that these sequences were entirely novel and had never been reported.

### Example 2 Establishing clonal lines reflecting properties of IMC-HM cells and IMC-LM cells

Clonal lines of IMC-HM cells and IMC-LM cells were established by the limiting dilution culture method. Cells of each strain were distributed on a 96-well plate to 4 cells/10 wells, and 10 clones that had each proliferated from a single cell were randomly selected.

In order to detect the expression of 23-1#2 and 28-1#3 in each of these clones, RT-PCR was performed as follows. The total RNA and the reverse transcriptase were prepared similarly to the above-described methods ((1) and (2) in Example 1). In order to amplify the specific cDNA by PCR, single-stranded oligo DNA primers, "23-1#2F1" (CAGAATCTGCTCATGCAGTC (SEQ ID NO: 29)), "23-1#2R1" (CACTCCTTACTTTCCACCCC (SEQ ID NO: 30)), "28-1#3F1" (AACAGCATTTCCCTAAAGCTCGG (SEQ ID NO: 31)), and "28-1#3R1" (TGGAAACTACTTCCCTGCTCCCA (SEQ ID NO: 32)) were designed.

Portions of the sequences of 23-1#2 and 28-1#3 can be specifically amplified using a set of 23-1#2F1 and 23-1#2R1 and another set of 28-1#3F1 and 28-1#3R1. Thirty cycles of PCR were performed using single-stranded cDNAs prepared from cells derived from IMC-HM and IMC-LM cells as a template, and EX Tag DNA polymerase under conditions similar to those in Example 1 (5). As a result, it was observed that both 23-1#2 and 28-1#3 were amplified as cDNAs of a similar appropriate size in the IMC-HM-derived clonal cells, while no signals were detected in the IMC-LM-derived clonal cells.

Next, in order to confirm the metastasis potency of each of the clonal cells *in vivo*, 5 x 10⁴ cells were subcutaneously transplanted to CDF1 mice. All clonal cells derived from IMC-HM killed the host two to three weeks after the transplantation, and their remarkable metastasis was observed with the naked eye in the liver and spleen while no metastasis foci were observed at all with the cells derived from IMC-LM. In addition, no distinct difference was detected between the cells derived from IMC-LM and those derived from IMC-HM, even by the observation of morphology, proliferation ability *in vitro*, etc. One clonal cell strain each was selected from the IMC-HM- and IMC-LM-derived clonal cells and designated "IMC-HA1" and "IMC-LE5," respectively. The IMC-HA1 cell was deposited with the following depositary institution:
(a) Name of the depositary institution and its address:
   Name: National Institute of Bioscience and Human-Technology Agency of Industrial Science and Technology
   Address: 1-3, Higashi 1-chome Tsukuba-shi Ibaraki-ken 305-0046 Japan
(b) Date of deposition (Date of original deposition): April 1, 1997
(c) Accession Number: FERM BP-6242

### Example 3 Analysis of clones 23-1#2 and 28-1#3 in IMC-HA1 and IMC-LE5 cells by Northern blot

In order to examine whether the expression of clones 23-1#2 and 28-1#3 differs at the poly(A) RNA level, Northern blot analysis was performed. Poly(A) RNA was prepared from IMC-HA1 and IMC-LE5 cells using a Messenger RNA Isolation Kit (STRATAGENE) as follows.

About 10⁸ cells were recovered from a culture suspension (100 ml) and dissolved in a denaturing solution (STRATAGENE) (5 ml) containing β -mercaptoethanol (50 µ l). An elution buffer (STRATAGENE) (10 ml) was added to this mixture, and the resulting mixture was centrifuged at 12,000 x g for 10 min. The supernatant was mixed with oligo-dT cellulose (0.04 g/ml) (5 ml) and gently stirred at room temperature for 15 min. After the mixture was centrifuged at 700 x g for 3 min, the supernatant was removed, and the cellulose was resuspended in a high-salt buffer (STRATAGENE) (5 ml). This washing process was repeated twice, and then the cellulose residue was suspended in a low-salt buffer (STRATAGENE) (5 ml). The cellulose suspension was then packed into a 2.5-ml push column (STRATAGENE), and the remaining poly(A) RNA was eluted with an elution buffer (400 µl) at 68°C. 3 M sodium acetate (40 µl) and 100% ethanol (1.1 ml) were added to the eluate, and the mixture was centrifuged at 15,000 x g. Precipitates thus obtained were rinsed with 80% ethanol and dissolved in a TE buffer containing no RNase (40 µl) to make a poly(A) RNA solution. Concentration was determined by measuring the absorbence (A260).

Next, for Northern blot analysis, 23-1#2 and 28-1#3 were directly radio-labelled with [α-³²P]dCTP using a Megaprime DNA labelling system (Amersham) according to the standard method. The primer (5 µl) was added to cDNA (100 ng) to make the total volume 33 µl, and the mixture was heated at 95°C for 5 min and allowed to stand at room temperature. A labelling buffer (Amersham) (10 µl), [α-³²P]dCTP (5 µl), and Klenow DNA polymerase I (1 unit/ µl) (2 µl) were added to this mixture , and the mixture was incubated at 37 °C for 10 min. To terminate the reaction, 0.5 M EDTA (3.5 µl) was added to this mixture , and the labelled probe and unreacted [α-³²P]dCTP were separated using a Sephadex G-50 spin column. Eluates from the column were diluted with purified water, and radioactivity of the probe was counted with a liquid scintillation counter.

Northern blot analysis was then performed. Poly(A) RNA (2 µ g) was loaded on a 1% agarose gel prepared in 1 x MOPS buffer and electrophoresed at 50 V for 1.5 hour. After the electrophoresis, the gel was left standing overnight to transfer the nucleic acid from the gel onto a Hybond N+ membrane (Amersham) via 10 x SSC buffer. After the transfer, the membrane was air-dried, and the nucleic acid on the membrane was fixed by UV with a STRATALINKER. After the UV treatment, hybridization was carried out as follows.

The membrane was soaked in a prehybridization solution (containing 5 x Denhardt's solution, 50% formamide, 2% SDS, and denatured salmon sperm DNA (100 µg/ml)), and prehybridization was performed at 42°C for 1 hour. [α-³²P]dCTP-labelled 23-1#2 and 28-1#3 were then added to the above-described prehybridization solution as the probe, and said membrane was allowed to stand at 42°C overnight. After the hybridization, the membrane was washed once with 2 x SSC in 0.1% SDS solution at 50°C for 30 min and twice with 0.5 x SSC in 0.1% SDS solution at 50°C for 30 min. The radioactivity was imaged according to the above-described method. 23-1#2 and 28-1#3 exhibited bands of about 1.1 kb and 0.7 kb, respectively, which hybridized only with the bands from IMC-HA1 Cells.

### Example 4 Analysis of clones 23-1#2 and 28-1#3 in IMC-HA1 and IMC-LE5 cells by Southern blot

Genomic DNA was prepared from IMC-HA1 and IMC-LE5 cells using a DNA Extraction Kit (STRATAGENE) and digested with EcoRI. The digested products were loaded (10 µg each) onto a 1% agarose gel prepared in a TAE buffer, separated by electrophoresis at 50 V, transferred to Hybond N+, and subjected to Southern blot analysis. Both cDNA probes exhibited identical band patterns. Therefore, it was indicated that both 23-1#2 and 28-1#3 were present in the genome of both IMC-HA1 and IMC-LE5 cells but expressed more predominantly in IMC-HA1 cells.

### Example 5 Cloning of full-length cDNAs of clones 23-1#2 and 28-1#3

### (1) Preparation of cDNA library derived from IMC-HA1 cells

A cDNA library derived from IMC-HA1 cells was prepared using a ZAP Express cDNA Synthesis Kit (STRATAGENE) by the following method.

### (a) Preparation of the total RNA and poly(A) RNA

The total RNA was extracted from IMC-HA1 cells by the, method described in Example 1 (1). In this case, extraction with ISOGEN was repeated twice to reduce the contamination of proteins, lipids, DNA fragments, etc. From this total RNA, poly(A) RNA was extracted as follows. A spin column was prepared by placing a Whatmann 3M filter paper on the bottom of a 2.5-ml syringe and filling it with 5 ml of oligo(dT)-cellulose (0.04 g/ml) (STRATAGENE). The column was loaded with a sample, allowed to stand at room temperature for 30 min, then washed three times with a high-salt buffer and a low-salt buffer (Clontech). After elution with an elution buffer (Clontech), a 1/5 volume of a sample buffer was added to the eluate,the resulting mixture was loadeded onto a new column, and the same procedure was repeated. After precipitation with ethanol and rinse, the product was dissolved in a TE buffer to make a poly(A) RNA solution.

### (b) Synthesis of the first strand cDNA

Poly(A) RNA (5 µg) and XhoI linker primer (170 pmol) were mixed, treated at 70°C for 5 min, then cooled in ice. 5 x first strand buffer (10 µl), 0.1 M dithiothreitol (GIBCO BRL) (5 µl), dNTP mix (10 mM each) (3 µl), and Rnasein (40 U/µl) (STRATAGENE) (1 µl) were added to this mixture , and reverse transcriptase Superscript II (GIBCO BRL) (12,000 U) was added to make a final volume of 50 µl. The resulting mixture was incubated at 44°C for 1 hour.

### (c) Synthesis of the second strand cDNA

A 10 x second strand buffer (20 µl), second strand dNTP mix (STRATAGENE) (6 µl), sterilized pure water (113.9 µl), and [α -³²P]dATP (Amersham) (2 µl) were added to the first strand cDNA synthesized above (45 µl), and *E. coli* RNase H (3 U) and *E. coli* DNA polymerase (STRATAGENE) (100 U) were added. The mixture was incubated at 16°C for 2.5 hours to synthesize the second strand.

### (d) Insertion of cDNA into the ZAP Express Vector

The free terminus of the XhoI linker primer was blunted with Pfu DNA polymerase (STRATAGENE) (5 U), ligated to the EcoRI adaptor with T4 DNA ligase (4 U) by allowing the mixture to stand at 8°C overnight, and further phosphorylated at the EcoRI terminus with T4 polynucleotide kinase (10 U). The XhoI linker terminus was cleaved by XhoI (120 U). cDNA was inserted into the ZAP Express Vector between the EcoRI and XhoI sites by reacting with the T4 DNA ligase at 12°C overnight.

### (e) In vitro packaging and titer assay

cDNA was packaged into phage using a Gigapack Gold II (STRATAGENE) exactly according to the process recommended by the supplier's protocol. The titer of the phage solution after packaging was assayed as follows. The original phage solution and its 10-fold dilution with an SM buffer were prepared separately, and 1 µl each of both solutions was incubated with 200 µl of XL1-Blue MRF' (OD₆₀₀ = 0.5) at 37°C for 15 min to infect the host bacteria. Incubated mixtures were overlaid on a 100 mm NZY agar (GIBCO) plate together with 3 ml of an LB top agar, 0.5 M IPTG, and 12.5 mg of X-Gal, and allowed to stand overnight. Ninety-five percent or more of plaques were found to retain the insert, and the volume of phage solution required for obtaining 50,000 plagues was determined from the number of plaques formed.

### (f) Amplification of cDNA library

Appropriate amounts of phage-infected host bacteria were overlaid on 20 previously prepared 150 mm NZY agar plates together with the top agar so that 50,000 plaques/plate could be obtained. These plates were then incubated at 37°C overnight to form plaques. After 8 ml of SM buffer was added, the plates were allowed to stand at 4°C overnight. The SM buffer in which phage was sufficiently released was recovered, and the plates were further rinsed with 2 ml of the same buffer to recover the phage. This was distributed in aliquots as the cDNA library stock solution and stored frozen at -70°C. This stock solution was used for screening the cDNA library .

### (2) Screening of cDNA library derived from IMC-HA1 cells

### (a) Primary screening

Twenty NZY agar plates were prepared in sterilized square #2 petri dishes. XL1-Blue MRF' adjusted to OD₆₀₀ = 0.5 was infected with the cDNA library phage so as to obtain 50,000 plaques/plate, overlaid on the above NZY agar plates together with an LB top agar, and allowed to stand at 37°C overnight. The next day, after suitable plaque formation was confirmed, the plates were cooled at 4°C for 2 hours, and DNA in plaques was transferred to a Hybond N+ membrane. The membrane was denatured, dried, UV cross-linked, then subjected to hybridization by the following procedure.

The membrane was soaked in a solution containing a 2 x PIPES buffer, 50% formamide, 0.5% SDS, and denatured salmon sperm DNA (100 µ g/ml) and prehybridized at 42 °C for 1 hour. [ α - ³²P]dCTP-labeled 23-1#2 and 28-1#3 were prepared according to the above-described method and added as probes to the prehybridization solution. The mixture was allowed to stand at 42°C overnight. After hybridization, the membrane was washed three to four times with a solution containing 0.1 x SSC and 0.1% SDS at 65°C for 20 min. The radioactivity was imaged according to the above-described method. Images were printed out in the actual size of the plate using a BAS 2000. Points corresponding to positive plaques on the plate were punched out with a sterilized and blunted tip of a P1000, and the phage was eluted into an SM buffer.

### (b) Secondary screening

Phage solutions that had been judged positive in the primary screening were further screened by a procedure similar to that described above. In this case, a serial 10-fold dilution of the phage solution was prepared and overlaid on 100 mm plastic dishes in place of sterilized square #2 petri dishes. The phages localized at the point judged positive in the secondary screening were eluted into the SM buffer, and the DNA insert was amplified using a set of T7 and T3 primers. At this stage, when many inserts were detected, a similar screening process was continued.

This screening of the cDNA library yielded a phage clone #9.3133 that specifically binds to 23-1#2, and clones #7.1641, #8.32, and #11.2441 that specifically bind to 28-1#3 were obtained.

### (c) Cloning into pBK-CMV phagemid vector

It is possible to directly excise out the ZAP Express vector *in vivo* as the pBK-CMV phagemid vector containing inserts in the presence of a helper phage (ExAssist helper phage). The pBK-CMV phagemid vector was excised out from the above-described phage clones, introduced into XLOLR cells, and allowed to form colonies on kanamycin-containing LB plates for cloning. Phagemid vector clones #9.31334, #7.16411, #8.323, and #11.24413 were thus obtained.

### (d) Sequencing of clones #9.31334, #7.16411, #8.323, and #11.24413

Partial sequencing of the clones was performed by the dye primer method using the pBK-CMV phagemid vector containing these cDNAs. Specifically; sequencing was performed similarly as described in Example 1 (6), except that the cycle sequencing reaction was carried out using T3 primer (AATTAACCCTCACTAAAGGG/SEQ ID NO: 33) or T7 primer (GTAATACGACTCACTATAGGGC/SEQ ID NO: 34). Furthermore, a primer to be used in RT-PCR and an appropriate primer were designed based on the sequence of the insert in the pBK-CMV phagemid vector whose cDNA sequence was partially determined by the dye primer method. The undetermined region was then sequenced by the dye terminator method using a PRISM Ready Reaction Terminator Cycle Sequencing Kit. Double-stranded DNA (250 to 500 ng), primer (3.2 pmol), and the above-described premix (8 µl) were mixed in a final volume of 20 µl, and the cycle sequencing was performed by the reaction of 96°C for 2 min, and 25 cycles of 96°C for 10 sec, 50°C for 5 sec, and 60°C for 4 min. Electrophoresis and analysis were performed under conditions similar to those in the dye primer method.

As a result, clone #9.31334 having the whole length of 983 bp (SEQ ID NO: 3) was obtained for 23-1#2. This sequence was found to be a novel gene comprising the region encoding 167 amino acids. This amino acid sequence exhibited about 38% homology to the bovine cystatin B, an inhibitory protein for cathepsins. Furthermore, three cDNA clones (#7.16411, #8.323, and #11.24413) were obtained for 28-1#3. Comparative studies on these nucleotide sequences revealed strong indications that #8.323 can be processed differently to produce #7.16411 and #11.24413. Clone #8.323 was 1,114 bp while clones #7.16411 and #11.24413 were 691 bp and 684 bp, respectively. Clones #7.16411 and #11.24413 represented an identical nucleotide sequence except for about 100 bp on the 5'-terminus side and were considered to encode the proteins of 131 and 126 amino acids with a difference of 20 to 30 amino acids at the N-terminus. From the deduced amino acid sequences, #7.16411 was considered to have a hydrophobic N-terminus, while #11.24413 was considered to be hydrophilic. A homology search indicated that three kinds of sequences obtained using 28-1#3 also represented novel genes similar to #9.31334. Furthermore, a protein presumed to be encoded by #7.16411 exhibited nearly 60% homology to human SLPI (secretoary leukoprotease inhibitor). Nucleotide sequences of these cDNA clones are shown in SEQ ID NO: 5 (nucleotide sequence of the clone #7.16411), SEQ ID NO: 7 (nucleotide sequence of the clone #8.323), and SEQ ID NO: 8 (nucleotide sequence of the clone #11.24413).

### Example 6 Expression of #7.16411 and #11.24413 in various cancer cells and normal tissues

Primers which specifically amplify #7.16411 and #11.24413 were designed, and the expression of these clones in various cancer cells and normal tissues was examined by RT-PCR. PCR was performed under the conditions in which each cDNA can be quantitatively detected in IMC-HA1 cells (conditions described below in Example 7). As set of primers, "7F2" (SEQ ID NO: 35) and "28-1#3F1" (SEQ ID NO: 31) were used to amplify #7.16411, and "11F2" (SEQ ID NO: 49) and "28-1#3F1" (SEQ ID NO: 31), to amplify #11.24413. As a result, it was found that #7.16411 was able to be amplified in almost all cancer cells and normal tissues examined, though the gradation of developed color differed somewhat. In contrast, #11.24413 could be detected only in cancer cells such as the P388 and IMC-HA1 cells, and in normal tissues such as the thymus, lung, and spleen, and slightly in peripheral blood. From these results, it is highly possible that the processing to produce #7.16411 is common in the majority of normal tissues, while #11.24413 is expressed only in particular cells. #11.24413 was considered to be an important factor to impart to the IMC-HA1 cells characteristics such as the ability to rapidly invade the whole body.

### Example 7 Expression of mouse CMAP in various cancer cell lines and normal tissues

The amino acid sequence deduced from the coding region of #9.31334 is designated mouse CMAP and is so called below.

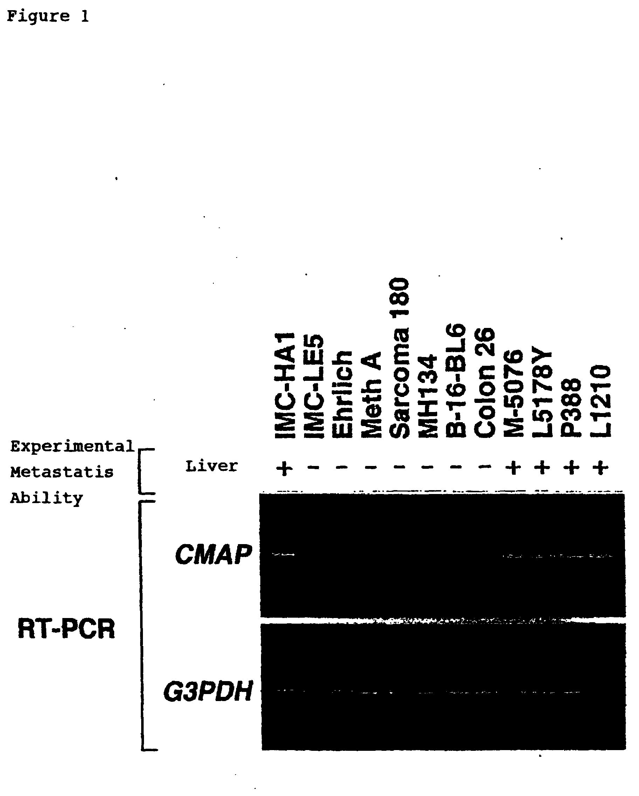

Expression of mouse CMAP was examined in 12 different mouse cancer cell lines by reverse transcription PCR (RT-PCR)(Figure 1). Total RNA was extracted from each cell line by a method similar to that in Example 1 (1), and a complementary single-stranded cDNA was prepared similarly to that in Example 1 (2). Mouse CMAP was then detected by PCR similarly as in Example 1 (5). The reaction was comprised of 94°C for 2 min; 30 cycles of 94°C for 1 min and 68°C for 2 min; and 68°C for 5 min, using a set of primers "23-1#2F1" (SEQ ID NO: 29) and "23-1#2R1" (SEQ ID NO: 30). Preliminary examination of the dilution of a single-stranded cDNA serving as a template confirmed that subquantitative conditions producing a nearly linear relationship between the dilution and product yield were established for IMC-HA1 cells when CMAP was diluted 10-fold for the amplification and the control G3PDH was diluted 40-fold for the detection.

Mouse CMAP was thus clearly expressed in M-5076 cells, L5178Y cells, P388 cells, and L1210 cells in addition to IMC-HA1 cells. Anatomical observation with the naked eye and pathological analysis confirmed that all of these cell lines formed metastatic foci mainly in the liver when 1 x 10⁵ cells were transplanted through the tail vein of mice. B-16-BL6 cells and Colon 26 cells in which almost no expression of mouse CMAP could be confirmed strongly induced an experimental metastatic foci in the lung, but not in the liver. Furthermore, all other cell lines wherein the expression of mouse CMAP was below the detection limit were almost rejected and regressed after the transplantation through the tail vein of mice. These results indicated that the expression of mouse CMAP could be closely associated with the metastasis of cancer cells to the liver.

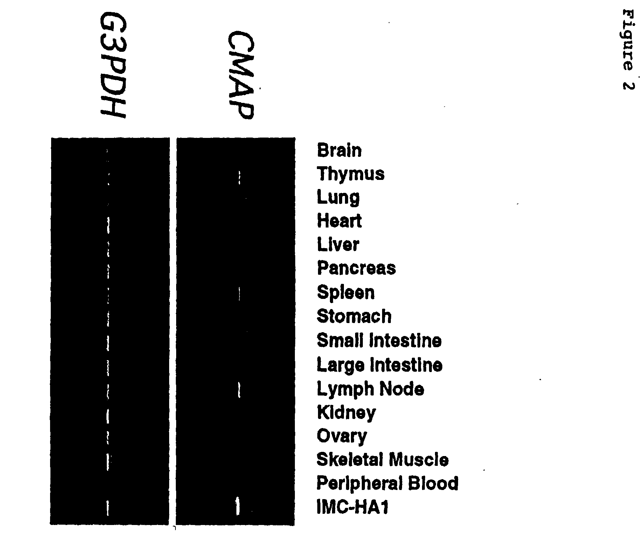

In addition, 15 tissues were surgically excised from two to three 5-week-old normal female CDF1 mice and disrupted using a Polytron homogenizer. The expression of mouse CMAP was confirmed by RT-PCR similarly as described above (Figure 2). Mouse CMAP was found to be expressed in the thymus, spleen, and mesenteric lymph nodes, although the expression was clearly weaker than for IMC-HA1 cells. These results indicated that mouse CMAP could also physiologically function in normal tissues, profoundly associating with the immune mechanism.

### Example 8 Identification of human homologue of mouse CMAP

### (1) Database Search

The present inventors searched for a human nucleotide sequence showing homology to that of mouse CMAP in the Genbank and EMBL databases with a Fast A using VMS (Version 8) software from Genetics Computer Group. The results revealed that two Expressed Sequence Tags (ESTs), N47763 and N56875, showed 75.7% and 72.0% homologies to mouse CMAP, respectively. These two ESTs hold a homologous sequence comprising 34 nucleotide residues, strongly indicating that they might be derived from human CMAP of the same origin.

### (2) Amplification of human CMAP by RT-PCR

Since mouse CMAP was expressed in the spleen among normal tissues of mice, the human spleen was chosen as the supply source of template DNA. A single-stranded cDNA was prepared from the total RNA (OriGene) (2 µg) extracted from the human spleen by a method similar to that in Example 1 (2). Based on sequences of N47763 and N56875, eight primers (SEQ ID NOs: H-CHAP-1 to 8) were designed. PCR was performed on the template single-stranded cDNA using two primers including H-CMAP-1 and H-CMAP-2 similarly as in Example 1 (5). Reaction was carried out in the sequence 94°C for 2 min; 30 cycles of 94°C for 30 sec, 60°C for 30 sec, and 72°C for 90 sec; and 72°C for 5 min. The thermal cycler used was a PERKIN ELMER 9600.

### (3) Sequencing

The nucleotide sequence of the cDNA product of about 0.8 kb (SEQ ID NO: 37) derived from the amplified human CMAP was directly sequenced according to a method similar to that in Example 1 (6) using primers H-CMAP-1 to 8 (SEQ ID NOs: 39 to 46). Human CMAP exhibited a 65.9% homology to mouse CMAP. In addition, human CMAP was located in the nucleotide residues from 83 to 583, and its amino acid sequence (SEQ ID NO: 38) possessed a 71.6% homology to that of mouse CMAP. Therefore, it was strongly indicated that these two proteins have approximately the same function.

### (4) Comparison of amino acid sequences of human CMAP with family 2 cystatin

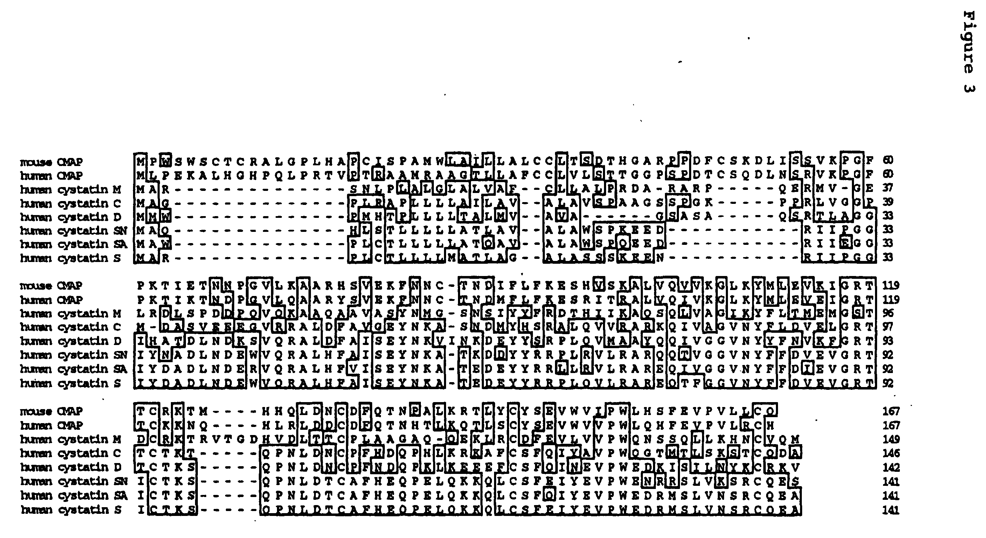

Amino acid sequences of mouse and human CMAPs and human family 2 cystatin were compared by a multi-sequence comparison program, MegAlign, in the Lasergene (DNASTAR) (Figure 3). Consensus sequences of human family 2 cystatin (cystein residues at. positions 121, 132, 146, and 166; glycine residue at 59; QXVXG at 103 to 107; and VFW at 153 to 155) were found to be minimally conserved in CMAPs. However, the overall homology was 30% or less in all CMAPs. In particular, sequences at the N-terminus were characteristic and long, and found to comprise many cysteine residues which may impart specificity to CMAP. Theoretically, it is highly possible that N of position 84 is glycosylated, a characteristic not observed in other sequences in its vicinity. Although CMAPs hold sequences resembling those hitheto reported for cystatins, they were found to comprise many characteristic amino acid residues, indicating the possibility of having properties differing from those of known substances.

### Example 9 Decrease in cancerous metastasis potency of IMC-HA1 cells in mice due to the constitutive decrease of mouse CMAP

It is generally agreed that alterations of the nature, quality or expression level of multiple overlapped genes are required for metastasis. Therefore, it was thought to be difficult to induce a single metastasis-associated gene in cells with low metastasis potency in mouse to confirm the elevation of their metastasis ability. IMC-HA1 cells were therefore transformed with a vector in which a sequence complementary to the mouse CMAP mRNA was inserted to construct stably transformed clonal cells, which were examined to determine the change in metastasis potency in mice.

### (1) Construction of pBK-AS-CMAP vector

The pBK-CMV expression vector was treated with restriction enzymes NheI and KpnI to prepare a linear vector whose lacZ promoter and multicloning sites were removed. The insert was prepared from the total mRNA of IMC-HA1 cells by RT-PCR as the starting material. CMAP-F1 (SEQ ID NO:. 47) and CHAP-R1 (SEQ ID NO: 48) were used as a set of primers. The NheI and KpnI restriction enzyme recognition sites are introduced in these primers at their termini, and their amplification product corresponds to the entire noncoding and coding regions on the 5'-side of mouse CMAP. The amplification product of about 0.5 kb was digested with restriction enzymes NheI and KpnI, purified with a QIAquick PCR Purification Kit (QIAGEN), and ligated to the above-prepared linear vector using a DNA Ligation Kit (Takara Shuzo). In this way, the pBK-AS-CMAP vector that expresses mRNA acting as the antisense against mouse CMAP in cells was constructed.

### (2) Transfer of pBK-AS-CMAP vector into IMC-HA1 cells and selection of transformants

After the pBK-AS-CMAP vector was introduced into *E. coli* competent cells and cultured on a large scale, plasmids were prepared in large amounts according to the standard method using a QIAfilter Plasmid Midi Kit (QIAGEN). The plasmid (about 50 µ g) was cleaved at one site with ApaLI to make it linear, purified by phenol extraction and ethanol precipitation, and dissolved in EP buffer (50 µl) (containing 25 mM Hepes (pH 7.0), 137 mM NaCl, 5 mM KCl, 0.27mM Na₂HPO₄, and 6mM dextrose). IMC-HA1 cells (about 1 x 10⁸ cells) at the logarithmic growth phase were recovered from the culture medium (100 ml) and resuspended in EP buffer to prepare a cell suspension of 4 x 10⁷ cells/ml. This cell suspension (450 µl) was mixed with the plasmid solution prepared above (50 µ 1), and plasmids were transfected into cells by electroporation under the conditions of 250 mV, 500 µF, and a single pulse using a GenePulser II (Bio-Rad). After the mixture was allowed to stand at room temperature for 10 min, cells were cultured under the usual conditions. After 24-hour culturing, transformed cells were continuously treated with neomycin (0.3 mg/ml) for 2 weeks to select stable transformants. The cells were cloned by the limiting dilution method to obtain about 50 clonal cells. The cells in which the expression level of mouse CMAP was decreased were screened by RT-PCR. As a result, three lines of cell clones, 57C4, 53A9, and 53E9, were obtained. Similar procedures were also performed with pBK-CMV vector with no insert, and an 18B5 cell line was obtained as the vector control cell clone. When the expression of mouse CMAP mRNA in these cell lines was confirmed by Northern blot analyses, said mRNA was expressed in the 18B5 cell line at almost the same level as that in the IMC-HA1 cell line, but only about 49%, 32%, and 16% in 57C4, 53A9, and 53E9 cell lines. No particular changes in the proliferation and morphology during culturing were noticed in any of these cell lines.

### (3) Confirmation of metastasis potency in vivo

Metastasis abilities of cloned cell lines derived from IMC-HA1 cells with different expression patterns of mouse CMAP mRNA were tested in mice by three independent experiment systems. In experiment I, each cell line (5 x 10⁴ cells) was subcutaneously transplanted to mice. The liver was excised on the 15th day after the transplantation and subjected to observation of appearance, weight determination, and calculation of the survival rate. Experiment II was performed in the same matter as experiment I except that on the third day after the transplantation primary tumors at the transplantation site were excised together with proximal lymph nodes. In this experiment, a model of a minute metastatic focus of IMC-HA1 cells in the liver can be prepared. Furthermore, in experiment III, cells (1 x 10³ cells) were directly transferred through the tail vein, and the liver was excised on the 13th day after transplantation. At the same time, a diachronic study of the survival was performed. The experiment revealed that the expression of mouse CMAP mRNA was closely associated with the formation of liver metastatic focus and survival of mice (Table 1). Note 1) in the table represents "Number of mice which survived for 60 days/Number of mice tested."

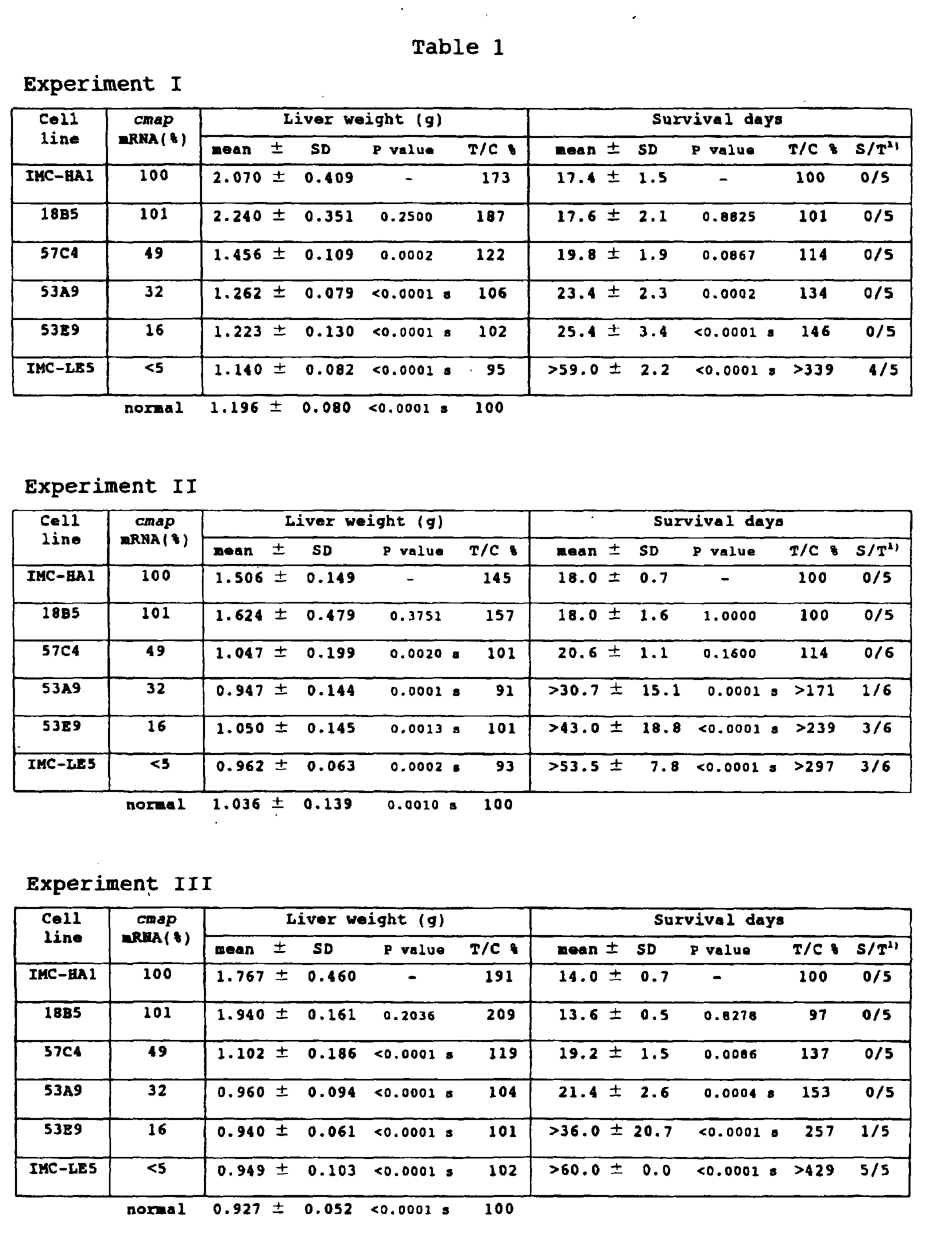

It is especially noteworthy that in experiment II, one out of six mice in which 53A9 cells were transplanted and three (half) of six mice in which 53E9 cells were transplanted survived for 60 days or more without metastasis to the liver. These results suggested that the expression of mouse CMAP is profoundly associated with the formation of metastatic focus in the liver, and that the gene acts mainly after cancer cells enter the vessel.

### Industrial Applicability

The present invention provides a novel protein associated with cancer metastasis and a DNA encoding said protein. This invention also provides a vector carrying said DNA, a transformant harboring said vector, and a method for preparing a recombinant protein by culturing said transformant. In addition, this invention provides DNA to be used to detect, isolate, amplify, or suppress the expression of said gene. Furthermore, this invention provides a method for screening cancer metastasis inhibitors utilizing said protein, enabling development of novel cancer metastasis inhibitors.

Sequence Listing

(1) Name of Applicant: Banyu Pharmaceutical Co., Ltd.

(2) Title of the Invention: Cancer Metastasis-associated Gene

(3) Reference Number: B1-001PCT

(4) Application Number:

(5) Filing Date:

(6) Country where the priority application was filed and the application number
of the application: Japan, No. Hei 9-105333

(7) Priority date: April 8, 1997

(8) Number of Sequences: 49

SEQ ID NO: 1
SEQUENCE LENGTH: 656
SEQUENCE TYPE: nucleic acid
STRANDEDNESS: double
TOPOLOGY: linear
MOLECULE TYPE: cDNA
HYPOTHETICAL: No
ANTISENSE: Yes
ORIGINAL SOURCE:
ORGANISM: Mus Musculus
CELL LINE: IMC-HA1 cell

SEQUENCE DESCRIPTION: SEQ ID NO: 1
TATTATGTTG AGTGATATTT TTTTTTTTAG GTGATCAGCT TTTTATTTAT GATAAATCAT 60
CCCCATCAAT ATTAGTCTGA TGGTAGCAGA CCAGACGGCT GCAGAATCTG CTCATGCAGT 120
CGGGCATCAA GAAGCTTCCC ACACTACCAC CTTGTTGACT GTATCTGCTT TGCTCTGCAC 180
GTGCTCCAGT AATGTCATCA AAGGACTCTC TGCGTGCCCA CATCCTGAAT CTAGGTGTGT 240

AGCACCAGAG AATTCATTTT AGAAGAAAGT GTGAATACAG TTAATCTGGA GAGGAGGGCA 300
CTATATTCTC CCAGTCCAGC CCCCTGATGA CCCACTGGCA TGTTAATCTG CTCGCTGGTA 360
AGCAGGCCCT GAGGATCTGA GATGGCCAGG ATGGGGTGGA AAGTAAGGAG TGGGGAAAAC 420
ACGAGTGGAT ATGTATCACA GCTGCAGTCT TGCTAAAGAG ACAGAAGTCA CTGGCAGAGG 480
AGAACAGGCA CCTCAAAACT GTGGAGCCAG GGGATGACCC AGACTTCAGA GTAGCAATAT 540
AGAGTCCGCT TCAAGGCAGG GTTGGTTTGG AAGTCACAGT TGTCCAGTTG GTGATGCATG 600
GTCTTCCTGC ATGTAGTTCG GCCGATTTTC ACCTCCAGCA TTTAGTGAGG GTTAAT 656

SEQ ID NO: 2
SEQUENCE LENGTH: 369
SEQUENCE TYPE: nucleic acid
STRANDEDNESS: double
TOPOLOGY: linear
MOLECULE TYPE: cDNA
HYPOTHETICAL: No
ANTISENSE: Yes
ORIGINAL SOURCE:
ORGANISM: Mus Musculus
CELL LINE: IMC-HA1 cell

SEQUENCE DESCRIPTION: SEQ ID NO: 2
CATTATGCTG AGTGATATCT TTTTTTTTGC ATAGAGAAAT GAATGCGTTT ATTTATTTGC 60
TCTCCAACAG CATTTCCCTA AAGCTCGGTG AATGCTGAGC CAAAAGGAGA TGTTAGTGGT 120
AAAACAGTAA CCCTGGTCCA CAGGATCCAA GCCTGGGATA CAGGGAGCCG GGAGCCATGG 180
AACCCGGAGC AGGGACGCCT GGGAGCAGGG AAGTAGTCTC CAGAGCACAC CGAGCACGAG 240
TCCAGAGCCG GCGCCAATGT CAGGGATCAG GCTCACATCG GGGGCAGGCA GACTTTCCCA 300
CATATACCCT CACAGCACTT GTATTTGCCG TCACACTGCC CGTCCCTCCA GCATTTAGTG 360
AGGGTTAAT 369

SEQ ID NO: 3
SEQUENCE LENGTH: 983
SEQUENCE TYPE: nucleic acid
STRANDEDNESS: double
TOPOLOGY : linear
MOLECULE TYPE: cDNA to mRNA
HYPOTHETICAL: No

ANTISENSE: No
ORIGINAL SOURCE:
ORGANISM: Mus Musculus
CELL LINE: IMC-HA1 cell
IMMEDIATE SOURCE
LIBRARY : IMC-HA1 cell-derived cDNA library
CLONE : 23-1#2
FEATURE:
NAME/KEY : CDS
LOCATION : 17..520
IDENTIFICATION METHOD: E

SEQUENCE DESCRIPTION: SEQ ID NO: 3
CTGAAGCTAC CCCACC ATG CCC TGG TCC TGG AGC TGT ACT TGC CGA GCA CTT 52
Met Pro Trp Ser Trp Ser Cys Thr Cys Arg Ala Leu
1               5                   10
GGC CCA CTA CAT GCT CCC TGC ATT TCC CCA GCC ATG TGG CTG GCC ATT 100
Gly Pro Leu His Ala Pro Cys Ile Ser Pro Ala Met Trp Leu Ala Ile
        15                  20                  25
CTG CTT GCC CTC TGC TGC CTA ACT TCT GAC ACC CAT GGG GCA CGC CCC 148
Leu Leu Ala Leu Cys Cys Leu Thr Ser Asp Thr His Gly Ala Arg Pro
    30                  35                  40
CCA GAT TTT TGT TCC AAA GAT TTG ATC TCC AGT GTG AAG CCA GGA TTC 196
Pro Asp Phe Cys Ser Lys Asp Leu Ile Ser Ser Val Lys Pro Gly Phe
45                  50                  55                  60
CCC AAA ACA ATA GAG ACC AAT AAC CCA GGA GTG CTT AAG GCC GCC AGG 244
Pro Lys Thr Ile Glu Thr Asn Asn Pro Gly Val Leu Lys Ala Ala Arg
                65                  70                  75
CAC AGT GTG GAA AAG TTC AAC AAC TGC ACA AAT GAC ATC TTT TTG TTC 292
His Ser Val Glu Lys Phe Asn Asn Cys Thr Asn Asp Ile Phe Leu Phe
            80                  85                  90
AAG GAG TCC CAT GTC AGC AAA GCC CTG GTA CAG GTG GTG AAA GGC CTG 340
Lys Glu Ser His Val Ser Lys Ala Leu Val Gln Val Val Lys Gly Leu
        95                  100                 105
AAA TAT ATG CTG GAG GTG AAA ATC GGC CGA ACT ACA TGC AGG AAG ACC 388
Lys Tyr Met Leu Glu Val Lys Ile Gly Arg Thr Thr Cys Arg Lys Thr

    110                 115                 120
ATG CAT CAC CAA CTG GAC AAC TGT GAC TTC CAA ACC AAC CCT GCC TTG 436
Met His His Gln Leu Asp Asn Cys Asp Phe Gln Thr Asn Pro Ala Leu
125                 130                 135                 140
AAG CGG ACT CTA TAT TGC TAC TCT GAA GTC TGG GTC ATC CCC TGG CTC 484
Lys Arg Thr Leu Tyr Cys Tyr Ser Glu Val Trp Val Ile Pro Trp Leu
                145                 150                 155
CAC AGT TTT GAG GTG CCT GTT CTC CTC TGC CAG TGACTTCTGT CTCTTTAGCA 537
His Ser Phe Glu Val Pro Val Leu Leu Cys Gln
            160                 165
AGACTGCAGC TGTGATACAT ATCCACTCGT GTTTTCCCCA CTCCTTACTT TCCACCCCAT 597
CCTGGCCATC TCAGATCCTC AGGGCCTGCT TACCAGCGAG CAGATTAACA TGCCAGTGGG 657
TCATCAGGGG GCTGGACTGG GAGAATATAG TGCCCTCCTC TCCAGATTAA CTGTATTCAC 717
ACTTTCTTCT AAAATGAATT CTCTGGTGCT ACACACCTAG ATTCAGGATG TGGGCACGCA 777
GAGAGTCCTT TGATGACATT ACTGGAGCAC GTGCAGAGCA AAGCAGATAC AGTCAACAAG 837
GTGGTAGTGT GGGAAGCTTC TTGATGCCCG ACTGCATGAG CAGATTCTGC AGCCGTCTGG 897
TATGCTACCA TCAGACTAAT ATTGATGGGG ATGATTTATC ATAAATAAAA AGCTGATCAC 957
CTAAAAGCAA AAAAAAAAAA AAAAAA 983

SEQ ID NO: 4
SEQUENCE LENGTH: 167
SEQUENCE TYPE: amino acid
TOPOLOGY: linear
MOLECULE TYPE: protein
ORIGINAL SOURCE:
ORGANISM: Mus Musculus
CELL LINE: IMC-HA1 cell

SEQUENCE DESCRIPTION: SEQ ID NO: 4
Met Pro Trp Ser Trp Ser Cys Thr Cys Arg Ala Leu
1               5                   10
Gly Pro Leu His Ala Pro Cys Ile Ser Pro Ala Met Trp Leu Ala Ile
        15                  20                  25
Leu Leu Ala Leu Cys Cys Leu Thr Ser Asp Thr His Gly Ala Arg Pro
    30                  35                  40
Pro Asp Phe Cys Ser Lys Asp Leu Ile Ser Ser Val Lys Pro Gly Phe

45                  50                  55                  60
Pro Lys Thr Ile Glu Thr Asn Asn Pro Gly Val Leu Lys Ala Ala Arg
                65                  70                  75
His Ser Val Glu Lys Phe Asn Asn Cys Thr Asn Asp Ile Phe Leu Phe
            80                  85                  90
Lys Glu Ser His Val Ser Lys Ala Leu Val Gln Val Val Lys Gly Leu
        95                  100                 105
Lys Tyr Met Leu Glu Val Lys Ile Gly Arg Thr Thr Cys Arg Lys Thr
    110                 115                 120
Met His His Gln Leu Asp Asn Cys Asp Phe Gln Thr Asn Pro Ala Leu
125                 130                 135                 140
Lys Arg Thr Leu Tyr Cys Tyr Ser Glu Val Trp Val Ile Pro Trp Leu
                145                 150                 155
His Ser Phe Glu Val Pro Val Leu Leu Cys Gln
            160                 165

SEQ ID NO: 5
SEQUENCE LENGTH: 691
SEQUENCE TYPE: nucleic acid
STRANDEDNESS: double
TOPOLOGY: linear
MOLECULE TYPE: cDNA to mRNA
HYPOTHETICAL: No
ANTISENSE: No
ORIGINAL SOURCE:
ORGANISM: Mus Musculus
CELL LINE: IMC-HA1 cell
IMMEDIATE SOURCE:
LIBRARY: IMC-HA1 cell-derived cDNA library
CLONE: 28-1#3
FEATURE:
NAME/KEY: CDS
LOCATION: 37..429
IDENTIFICATION METHOD: E

SEQUENCE DESCRIPTION: SEQ ID NO: 5

CTCGGGACTG GTCATCAGAG CTCCCCTGCC TTCACC ATG AAG TCC TGC GGC CTT 54
Met Lys Ser Cys Gly Leu
1               5
TTA CCT TTC ACG GTG CTC CTT GCT CTG GGG ATC CTG GCA CCC TGG ACT 102
Leu Pro Phe Thr Val Leu Leu Ala Leu Gly Ile Leu Ala Pro Trp Thr
            10                  15                  20
GTG GAA GGA GGC AAA AAT GAT GCT ATC AAA ATC GGA GCC TGC CCT GCT 150
Val Glu Gly Gly Lys Asn Asp Ala Ile Lys Ile Gly Ala Cys Pro Ala
        25                  30                  35
AAA AAG CCT GCC CAG TGC CTT AAG CTT GAG AAG CCA CAA TGC CGT ACT 198
Lys Lys Pro Ala Gln Cys Leu Lys Leu Glu Lys Pro Gln Cys Arg Thr
    40                  45                  50
GAC TGG GAG TGC CCG GGA AAG CAG AGG TGC TGC CAA GAT GCT TGC GGT 246
Asp Trp Glu Cys Pro Gly Lys Gln Arg Cys Cys Gln Asp Ala Cys Gly
55                  60                  65                  70
TCC AAG TGC GTG AAT CCT GTT CCC ATT CGC AAA CCA GTG TGG AGG AAG 294
Ser Lys Cys Val Asn Pro Val Pro Ile Arg Lys Pro Val Trp Arg Lys
                75                  80                  85
CCT GGG AGG TGC GTC AAA ACT CAG GCA AGA TGT ATG ATG CTT AAC CCT 342
Pro Gly Arg Cys Val Lys Thr Gln Ala Arg Cys Met Met Leu Asn Pro
            90                  95                  100
CCC AAT GTC TGC CAG AGG GAC GGG CAG TGT GAC GGC AAA TAC AAG TGC 390
Pro Asn Val Cys Gln Arg Asp Gly Gln Cys Asp Gly Lys Tyr Lys Cys
        105                 110                 115
TGT GAG GGT ATA TGT GGG AAA GTC TGC CTG CCC CCG ATG TGAGCCTGAT 439
Cys Glu Gly Ile Cys Gly Lys Val Cys Leu Pro Pro Met
    120                 125                 130
CCCTGACATT GGCGCCGGCT CTGGACTCGT GCTCGGTGTG CTCTGGAAAC TACTTCCCTG 499
CTCCCAGGCG TCCCTGCTCC GGGTTCCATG GCTCCCGGCT CCCTGTATCC CAGGCTTGGA 559
TCCTGTGGAC CAGGGTTACT GTTTTACCAC TAACATCTCC TTTTGGCTCA GCATTCACCG 619
AGCTTTAGGG AAATGCTGTT GGAGAGCAAA TAAATAAACG CATTCATTTC TCTAAAAAAA 679
AAAAAAAAAA AA 691

SEQ ID NO: 6
SEQUENCE LENGTH: 131
SEQUENCE TYPE: amino acid

TOPOLOGY: linear
MOLECULE TYPE: protein
ORIGINAL SOURCE:
ORGANISM: Mus Musculus
CELL LINE: IMC-HA1 cell
SEQUENCE DESCRIPTION: SEQ ID NO: 6
Met Lys Ser Cys Gly Leu
1               5
Leu Pro Phe Thr Val Leu Leu Ala Leu Gly Ile Leu Ala Pro Trp Thr
            10                  15                  20
Val Glu Gly Gly Lys Asn Asp Ala Ile Lys Ile Gly Ala Cys Pro Ala
        25                  30                  35
Lys Lys Pro Ala Gln Cys Leu Lys Leu Glu Lys Pro Gln Cys Arg Thr
    40                  45                  50
Asp Trp Glu Cys Pro Gly Lys Gln Arg Cys Cys Gln Asp Ala Cys Gly
55                  60                  65                  70
Ser Lys Cys Val Asn Pro Val Pro Ile Arg Lys Pro Val Trp Arg Lys
                75                  80                  85
Pro Gly Arg Cys Val Lys Thr Gln Ala Arg Cys Met Met Leu Asn Pro
            90                  95                  100
Pro Asn Val Cys Gln Arg Asp Gly Gln Cys Asp Gly Lys Tyr Lys Cys
        105                 110                 115
Cys Glu Gly Ile Cys Gly Lys Val Cys Leu Pro Pro Met
    120                 125                 130

SEQ ID NO: 7
SEQUENCE LENGTH: 1114
SEQUENCE TYPE: nucleic acid
STRANDEDNESS: double
TOPOLOGY: linear
MOLECULE TYPE: cDNA
HYPOTHETICAL: No
ANTISENSE: No
ORIGINAL SOURCE:
ORGANISM: Mus Musculus
CELL LINE: IMC-HA1 cell

IMMEDIATE SOURCE:
LIBRARY: IMC-HA1 cell-derived cDNA library
CLONE: #8.323

SEQUENCE DESCRIPTION: SEQ ID NO: 7
CCAGAGGCCA GTTTGGGATG CCAAACCCCT ACCTAACCAG AAGAAGAGAA GAAAGGCCAC 60
TGCCGAGGTC ACTTCCAGTA CTTGGAGGAG AAAGCAACGT TCCCATTTAC AGCTGAGTAA 120
CAGGAGCCAC AAGGTATGTC TGACTCAAAA GTTCAGGCTC TCGATGACTG TGCGGTGCTG 180
CCCAGTGTGT CTTCTTCAAT GTAACCTCAG GACCTAGAAC AGCACCTTGC ATGTGCTCTC 240
AGGTGGTTAC TCTGATGGCC TCATGGTCCT GCCTGAAACA GAAAGTCTGC CACCTACTTC 300
TGTAGCAGCA AGACTCCTGT TCTGTGGCTA AGCTTCCTGC CTGTGCAAGA GCCACAGGGA 360
GGGGCCAAAT GCATGCCACT GGGGCCACGC CTCCTGGTAA AGACATAAAT AGTGATCCTC 420
GGGACTGGTC ATCAGAGCTC CCCTGCCTTC ACCATGAAGT CCTGCGGCCT TTTACCTTTC 480
ACGGTGCTCC TTGCTCTGGG GATCCTGGCA CCCTGGACTG TGGAAGGAGG CAAAAATGAT 540
GCTATCAAAA TCGGAGCCTG CCCTGCTAAA AAGCCTGCCC AGTGCCTTAA GCTTGAGAAG 600
CCACAATGCC GTACTGACTG GGAGTGCCCG GGAAAGCAGA GGTGCTGCCA AGATGCTTGC 660
GGTTCCAAGT GCGTGAATCC TGTTCCCATT CGCAAACCAG TGTGGAGGAA GCCTGGGAGG 720
TGCGTCAAAA CTCAGGCAAG ATGTATGATG CTTAACCCTC CCAATGTCTG CCAGAGGGAC 780
GGGCAGTGTG ACGGCAAATA CAAGTGCTGT GAGGGTATAT GTGGGAAAGT CTGCCTGCCC 840
CCGATGTGAG CCTGATCCCT GACATTGGCG CCGGCTCTGG ACTCGTGCTC GGTGTGCTCT 900
GGAAACTACT TCCCTGCTCC CAGGCGTCCC TGCTCCGGGT TCCATGGCTC CCGGCTCCCT 960
GTATCCCAGG CTTGGATCCT GTGGACCAGG GTTACTGTTT TACCACTAAC ATCTCCTTTT 1020
GGCTCAGCAT TCACCGAGCT TTAGGGAAAT GCTGTTGGAG AGCAAATAAA TAAACGCATT 1080
CATTTCTCTA TGCAAAAAAA AAAAAAAAAA AAAA 1114

SEQ ID NO: 8
SEQUENCE LENGTH: 684
SEQUENCE TYPE: nucleic acid
STRANDEDNESS: double
TOPOLOGY: linear
MOLECULE TYPE: cDNA to mRNA
HYPOTHETICAL: No
ANTISENSE: No
ORIGINAL SOURCE:
ORGANISM: Mus Musculus
CELL LINE: IMC-HA1 cell

ORIGINAL SOURCE:
LIBRARY: IMC-HA1 cell-derived cDNA library
CLONE: 28-1#3
FEATURE:
NAME/KEY: CDS
LOCATION: 40..420
IDENTIFICATION METHOD: E

SEQUENCE DESCRIPTION: SEQ ID NO: 8
TGTTATTAAT GATCTGGAAG CACCAGAGGC CAGTTTGGG ATG CCA AAC CCC TAC CTA 57
Met Pro Asn Pro Tyr Leu
1               5
ACC AGA AGA AGA GAA GAA AGG CCA CTG CCG AGG TCA CTT CCA GTA CTT 105
Thr Arg Arg Arg Glu Glu Arg Pro Leu Pro Arg Ser Leu Pro Val Leu
            10                  15                  20
GGA GAT GCT ATC AAA ATC GGA GCC TGC CCT GCT AAA AAG CCT GCC CAG 153
Gly Asp Ala Ile Lys Ile Gly Ala Cys Pro Ala Lys Lys Pro Ala Gln
        25                  30                  35
TGC CTT AAG CTT GAG AAG CCA CAA TGC CGT ACT GAC TGG GAG TGC CCG 201
Cys Leu Lys Leu Glu Lys Pro Gln Cys Arg Thr Asp Trp Glu Cys Pro
    40                  45                  50
GGA AAG CAG AGG TGC TGC CAA GAT GCT TGC GGT TCC AAG TGC GTG AAT 249
Gly Lys Gln Arg Cys Cys Gln Asp Ala Cys Gly Ser Lys Cys Val Asn
55                  60                  65                  70
CCT GTT CCC ATT CGC AAA CCA GTG TGG AGG AAG CCT GGG AGG TGC GTC 297
Pro Val Pro Ile Arg Lys Pro Val Trp Arg Lys Pro Gly Arg Cys Val
                75                  80                  85
AAA ACT CAG GCA AGA TGT ATG ATG CTT AAC CCT CCC AAT GTC TGC CAG 345
Lys Thr Gln Ala Arg Cys Met Met Leu Asn Pro Pro Asn Val Cys Gln
            90                  95                  100
AGG GAC GGG CAG TGT GAC GGC AAA TAC AAG TGC TGT GAG GGT ATA TGT 393
Arg Asp Gly Gln Cys Asp Gly Lys Tyr Lys Cys Cys Glu Gly Ile Cys
        105                 110                 115
GGG AAA GTC TGC CTG CCC CCG ATG TGAGCCTGAT CCCTGACATT GGCGCCGGCT 447
Gly Lys Val Cys Leu Pro Pro Met
    120                 125

CTGGACTCGT GCTCGGTGTG CTCTGGAAAC TACTTCCCTG CTCCCAGGCG TCCCTGCTCC 507
GGGTTCCATG GCTCCCGGCT CCCTGTATCC CAGGCTTGGA TCCTGTGGAC CAGGGTTACT 567
GTTTTACCAC TAACATCTCC TTTTGGCTCA GCATTCACCG AGCTTTAGGG AAATGCTGTT 627
GGAGAGCAAA TAAATAAACG CATTCATTTC TCTATGCAAA AAAAAAAAAA AAAAAAA 684

SEQ ID NO: 9
SEQUENCE LENGTH: 126
SEQUENCE TYPE: amino acid
TOPOLOGY: linear
MOLECULE TYPE: protein
ORIGINAL SOURCE:
ORGANISM: Mus Musculus
CELL LINE: IMC-HA1 cell

SEQUENCE DESCRIPTION: SEQ ID NO: 9
Met Pro Asn Pro Tyr Leu
1               5
Thr Arg Arg Arg Glu Glu Arg Pro Leu Pro Arg Ser Leu Pro Val Leu
            10                  15                  20
Gly Asp Ala Ile Lys Ile Gly Ala Cys Pro Ala Lys Lys Pro Ala Gln
        25                  30                  35
Cys Leu Lys Leu Glu Lys Pro Gln Cys Arg Thr Asp Trp Glu Cys Pro
    40                  45                  50
Gly Lys Gln Arg Cys Cys Gln Asp Ala Cys Gly Ser Lys Cys Val Asn
55                  60                  65                  70
Pro Val Pro Ile Arg Lys Pro Val Trp Arg Lys Pro Gly Arg Cys Val
                75                  80                  85
Lys Thr Gln Ala Arg Cys Met Met Leu Asn Pro Pro Asn Val Cys Gln
            90                  95                  100
Arg Asp Gly Gln Cys Asp Gly Lys Tyr Lys Cys Cys Glu Gly Ile Cys
        105                 110                 115
Gly Lys Val Cys Leu Pro Pro Met
    120                 125

SEQ ID NO: 10
SEQUENCE LENGTH: 25

SEQUENCE TYPE: nucleic acid
STRANDEDNESS: single
TOPOLOGY: linear
MOLECULE TYPE: synthetic DNA
SEQUENCE DESCRIPTION: SEQ ID NO: 10
ATTAACCCTC ACTAAATGCT GGGGA 25

SEQ ID NO: 11
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION: SEQ ID NO: 11
ATTAACCCTC ACTAAATGCT GGAGG 25

SEQ ID NO: 12
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION: SEQ ID NO: 12
ATTAACCCTC ACTAAATGCT GGTGG 25

SEQ ID NO: 13
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION: SEQ ID NO: 13
ATTAACCCTC ACTAAATGCT GGTAG 25

SEQ ID NO: 14
SEQUENCE LENGTH: 26

SEQUENCE TYPE: nucleic acid
STRANDEDNESS: single
TOPOLOGY: linear
MOLECULE TYPE: synthetic DNA
SEQUENCE DESCRIPTION: SEQ ID NO: 14
ATTAACCCTC ACTAAAGATC TGACTG 26

SEQ ID NO: 15
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION: SEQ ID NO: 15
ATTAACCCTC ACTAAATGCT GGGTG 25

SEQ ID NO: 16
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION: SEQ ID NO: 16
ATTAACCCTC ACTAAATGCT GTATG 25

SEQ ID NO: 17
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION: SEQ ID NO: 17
ATTAACCCTC ACTAAATGGA GCTGG 25

SEQ ID NO: 18
SEQUENCE LENGTH: 25

SEQUENCE TYPE: nucleic acid
STRANDEDNESS: single
TOPOLOGY: linear
MOLECULE TYPE: synthetic DNA
SEQUENCE DESCRIPTION: SEQ ID NO: 18
ATTAACCCTC ACTAAATGTG GCAGG 25

SEQ ID NO: 19
SEQUENCE LENGTH: 25
SEQUENCE TYPE: nucleic acid
STRANDEDNESS: single
TOPOLOGY: linear
MOLECULE TYPE: synthetic DNA
SEQUENCE DESCRIPTION: SEQ ID NO: 19
ATTAACCCTC ACTAAAGCAC CGTCC 25

SEQ ID NO: 20
SEQUENCE LENGTH: 30
SEQUENCE TYPE: nucleic acid
STRANDEDNESS: single
TOPOLOGY: linear
MOLECULE TYPE: synthetic DNA
SEQUENCE DESCRIPTION: SEQ ID NO: 20
CATTATGCTG AGTGATATCT TTTTTTTTAA 30

SEQ ID NO: 21
SEQUENCE LENGTH: 30
SEQUENCE TYPE: nucleic acid
STRANDEDNESS: single
TOPOLOGY: linear
MOLECULE TYPE: synthetic DNA
SEQUENCE DESCRIPTION: SEQ ID NO: 21
CATTATGCTG AGTGATATCT TTTTTTTTAC 30

SEQ ID NO: 22
SEQUENCE LENGTH: 30

STRANDEDNESS: single
TOPOLOGY: linear
MOLECULE TYPE: synthetic DNA
SEQUENCE DESCRIPTION: SEQ ID NO: 22
CATTATGCTG AGTGATATCT TTTTTTTTAG                                        30

SEQ ID NO: 23
SEQUENCE LENGTH: 30
SEQUENCE TYPE: nucleic acid
STRANDEDNESS: single
TOPOLOGY: linear
MOLECULE TYPE: synthetic DNA
SEQUENCE DESCRIPTION: SEQ ID NO: 23
CATTATGCTG AGTGATATCT TTTTTTTTCA                                        30

SEQ ID NO: 24
SEQUENCE LENGTH: 30
SEQUENCE TYPE: nucleic acid
STRANDEDNESS: single
TOPOLOGY: linear
MOLECULE TYPE: synthetic DNA
SEQUENCE DESCRIPTION: SEQ ID NO: 24
CATTATGCTG AGTGATATCT TTTTTTTTCC                                        30

SEQ ID NO: 25
SEQUENCE LENGTH: 30
SEQUENCE TYPE: nucleic acid
STRANDEDNESS: single
TOPOLOGY: linear
MOLECULE TYPE: synthetic DNA
SEQUENCE DESCRIPTION: SEQ ID NO: 25
CATTATGCTG AGTGATATCT TTTTTTTTCG                                        30

SEQ ID NO: 26
SEQUENCE LENGTH: 30
STRANDEDNESS: single

TOPOLOGY: linear
MOLECULE TYPE: synthetic DNA
SEQUENCE DESCRIPTION: SEQ ID NO: 26
CATTATGCTG AGTGATATCT TTTTTTTTGA 30

SEQ ID NO: 27
SEQUENCE LENGTH: 30
SEQUENCE TYPE: nucleic acid
STRANDEDNESS: single
TOPOLOGY: linear
MOLECULE TYPE: synthetic DNA
SEQUENCE DESCRIPTION: SEQ ID NO: 27
CATTATGCTG AGTGATATCT TTTTTTTGC 30

SEQ ID NO: 28
SEQUENCE LENGTH: 30
SEQUENCE TYPE: nucleic acid
STRANDEDNESS: single
TOPOLOGY: linear
MOLECULE TYPE: synthetic DNA
SEQUENCE DESCRIPTION: SEQ ID NO: 28
CATTATGCTG AGTGATATCT TTTTTTTGG 30

SEQ ID NO: 29
SEQUENCE LENGTH: 20
SEQUENCE TYPE: nucleic acid
STRANDEDNESS: single
TOPOLOGY: linear
MOLECULE TYPE: synthetic DNA
SEQUENCE DESCRIPTION: SEQ ID NO: 29
CAGAATCTGC TCATGCAGTC 20

SEQ ID NO: 30
SEQUENCE LENGTH: 20
SEQUENCE TYPE: nucleic acid
STRANDEDNESS: single

TOPOLOGY: linear
MOLECULE TYPE: synthetic DNA
SEQUENCE DESCRIPTION: SEQ ID NO: 30
CACTCCTTAC TTTCCACCCC 20

SEQ ID NO: 31
SEQUENCE LENGTH: 23
SEQUENCE TYPE: nucleic acid
STRANDEDNESS: single
TOPOLOGY: linear
MOLECULE TYPE: synthetic DNA
SEQUENCE DESCRIPTION: SEQ ID NO: 31
AACAGCATTT CCCTAAAGCT CGG 23

SEQ ID NO: 32
SEQUENCE LENGTH: 23
SEQUENCE TYPE: nucleic acid
STRANDEDNESS: single
TOPOLOGY: linear
MOLECULE TYPE: synthetic DNA
SEQUENCE DESCRIPTION: SEQ ID NO: 32
TGGAAACTAC TTCCCTGCTC CCA 23

SEQ ID NO: 33
SEQUENCE LENGTH: 20
SEQUENCE TYPE: nucleic acid
STRANDEDNESS: single
TOPOLOGY: linear
MOLECULE TYPE: synthetic DNA
SEQUENCE DESCRIPTION: SEQ ID NO: 33
AATTAACCCT CACTAAAGGG 20

SEQ ID NO: 34
SEQUENCE LENGTH: 22
SEQUENCE TYPE: nucleic acid
STRANDEDNESS: single

TOPOLOGY: linear
MOLECULE TYPE: synthetic DNA
SEQUENCE DESCRIPTION: SEQ ID NO: 34
GTAATACGAC TCACTATAGG GC 22

SEQ ID NO: 35
SEQUENCE LENGTH: 26
SEQUENCE TYPE: nucleic acid
STRANDEDNESS: single
TOPOLOGY: linear
MOLECULE TYPE: synthetic DNA
7F2
SEQUENCE DESCRIPTION: SEQ ID NO: 35
CTCGGGACTG GTCATCAGAG CTCCCC 26

SEQ ID NO: 36
SEQUENCE LENGTH: 24
SEQUENCE TYPE: nucleic acid
STRANDEDNESS: single
TOPOLOGY: linear
MOLECULE TYPE: synthetic DNA
SEQUENCE DESCRIPTION: SEQ ID NO: 37
GACTGTATCT GCTTTGCTCT GCAC 24

SEQ ID NO: 37
SEQUENCE LENGTH: 800
SEQUENCE TYPE: nucleic acid
STRANDEDNESS: double
TOPOLOGY: linear
MOLECULE TYPE: cDNA to mRNA
HYPOTHETICAL:
ANTISENSE: No
ORIGINAL SOURCE:
ORGANISM: Mus Muscukus
CELL LINE: IMC-HA1 cell
FEATURE:

NAME/KEY: CDS
LOCATION: 83..583
IDENTIFICATION METHOD : E

SEQUENCE DESCRIPTION: SEQ ID NO: 37
ACAGACCACT GCCCCCACCT GCCCTGCGCC ATCTACCCAA GAAGGCTCGG CACGGGCACC 60
AACCACTGCC TCCAACTGCC CC ATG CTG CCT GAG AAG GCA CTG CAC GGC CAC 112
Met Leu Pro Glu Lys Ala Leu His Gly His
1               5                   10
CCC CAA CTG CCC CGC ACT GTC CCT ACC CGG GCA GCC ATG CGA GCG GCT 160
Pro Gln Leu Pro Arg Thr Val Pro Thr Arg Ala Ala Met Arg Ala Ala
                15                  20                  25
GGA ACT CTG CTG GCC TTC TGC TGC CTG GTC TTG AGC ACC ACT GGG GGC 208
Gly Thr Leu Leu Ala Phe Cys Cys Leu Val Leu Ser Thr Thr Gly Gly
            30                  35                  40
CCT TCC CCA GAT ACT TGT TCC CAG GAC CTT AAC TCA CGT GTG AAG CCA 256
Pro Ser Pro Asp Thr Cys Ser Gln Asp Leu Asn Ser Arg Val Lys Pro
        45                  50                  55
GGA TTT CCT AAA ACA ATA AAG ACC AAT GAC CCA GGA GTC CTC CAA GCA 304
Gly Phe Pro Lys Thr Ile Lys Thr Asn Asp Pro Gly Val Leu Gln Ala
    60                  65                  70
GCC AGA TAC AGT GTT GAA AAG TTC AAC AAC TGC ACG AAC GAC ATG TTC 352
Ala Arg Tyr Ser Val Glu Lys Phe Asn Asn Cys Thr Asn Asp Met Phe
75                  80                  85                  90
TTG TTC AAG GAG TCC CGC ATC ACA AGG GCC CTA GTT CAG ATA GTG AAA 400
Leu Phe Lys Glu Ser Arg Ile Thr Arg Ala Leu Val Gln Ile Val Lys
                95                  100                 105
GGC CTG AAA TAT ATG CTC GAG GTG GAA ATT GGC AGA ACT ACC TGC AAG 448
Gly Leu Lys Thr Met Leu Glu Val Glu Ile Gly Arg Thr Thr Cys Lys
            110                 115                 120
AAA AAC CAG CAC CTG CGT CTG GAT GAC TGT GAC TTC CAA ACC AAC CAC 496
Lys Asn Gln His Leu Arg Leu Asp Asp Cys Asp Phe Gln Thr Asn His
        125                 130                 135
ACC TTG AAG CAG ACT CTG AGC TGC TAC TCT GAA GTC TGG GTC GTG CCC 544
Thr Leu Lys Gln Thr Leu Ser Cys Tyr Ser Glu Val Trp Val Val Pro
    140                 145                 150

TGG CTC CAG CAC TTC GAG GTG CCT GTT CTC CGT TGT CAC TGACCCCCGC 593
Trp Leu Gln His Phe Glu Val Pro Val Leu Arg Cys His
155                 160                 165
CTCTTCAGCA AGACCACAGC CATGACAAAC ACCAGGATGC ATGCTCCTTG TCCCCTCCCA 653
CCCGCCTCAT GACCCAGCCT CACAGACCCT CTCAGGCCTC TGACGAGTGA GCGGGTGAAG 713
TGCCACTGGG TCACCGCAGG GCAGCTGGAA TGGCAGCATG GTAGCGCCTC CTAACAGATT 773
AAATAGATCA CATTTGCTTC TAAAATT 800

SEQ ID NO: 38
SEQUENCE LENGTH: 167
SEQUENCE TYPE: amino acid
TOPOLOGY: linear
MOLECULE TYPE: protein
ORIGINAL SOURCE:
ORGANISM: Mus Muscukus
CELL LINE: IMC-HA1 cell

SEQUENCE DESCRIPTION: SEQ ID NO: 38
Met Leu Pro Glu Lys Ala Leu His Gly His
1               5                   10
Pro Gln Leu Pro Arg Thr Val Pro Thr Arg Ala Ala Met Arg Ala Ala
                15                  20                  25
Gly Thr Leu Leu Ala Phe Cys Cys Leu Val Leu Ser Thr Thr Gly Gly
            30                  35                  40
Pro Ser Pro Asp Thr Cys Ser Gln Asp Leu Asn Ser Arg Val Lys Pro
        45                  50                  55
Gly Phe Pro Lys Thr Ile Lys Thr Asn Asp Pro Gly Val Leu Gln Ala
    60                  65                  70
Ala Arg Tyr Ser Val Glu Lys Phe Asn Asn Cys Thr Asn Asp Met Phe
75                  80                  85                  90
Leu Phe Lys Glu Ser Arg Ile Thr Arg Ala Leu Val Gln Ile Val Lys
                95                  100                 105
Gly Leu Lys Thr Met Leu Glu Val Glu Ile Gly Arg Thr Thr Cys Lys
            110                 115                 120
Lys Asn Gln His Leu Arg Leu Asp Asp Cys Asp Phe Gln Thr Asn His
        125                 130                 135

Thr Leu Lys Gln Thr Leu Ser Cys Tyr Ser Glu Val Trp Val Val Pro
    140                 145                 150
Trp Leu Gln His Phe Glu Val Pro Val Leu Arg Cys His
155                 160                 165

SEQ ID NO: 39
SEQUENCE LENGTH: 21
SEQUENCE TYPE: nucleic acid
STRANDEDNESS: single
TOPOLOGY: linear
MOLECULE TYPE: synthetic DNA
SEQUENCE DESCRIPTION: SEQ ID NO: 39
ACAGACACTG CCCCCACCTG C 21

SEQ ID NO: 40
SEQUENCE LENGTH: 22
SEQUENCE TYPE: nucleic acid
STRANDEDNESS: single
TOPOLOGY: linear
MOLECULE TYPE: synthetic DNA
SEQUENCE DESCRIPTION: SEQ ID NO: 40
AATTTTAGAA GCAAATGTGA TC 22

SEQ ID NO: 41
SEQUENCE LENGTH: 23
SEQUENCE TYPE: nucleic acid
STRANDEDNESS: single
TOPOLOGY: linear
MOLECULE TYPE: synthetic DNA
SEQUENCE DESCRIPTION: SEQ ID NO: 41
ACATGTCGTT CGTGCAGTTG TTG 23

SEQ ID NO: 42
SEQUENCE LENGTH: 22
SEQUENCE TYPE: nucleic acid
STRANDEDNESS: single

TOPOLOGY: linear
MOLECULE TYPE: synthetic DNA
SEQUENCE DESCRIPTION: SEQ ID NO: 42
AATTGGCAGA ACTACCTGCA AG 22

SEQ ID NO: 43
SEQUENCE LENGTH: 22
SEQUENCE TYPE: nucleic acid
STRANDEDNESS: single
TOPOLOGY: linear
MOLECULE TYPE: synthetic DNA
SEQUENCE DESCRIPTION: SEQ ID NO: 43
GGTCTTGCTG AAGAGGCGGG GG 22

SEQ ID NO: 44
SEQUENCE LENGTH: 22
SEQUENCE TYPE: nucleic acid
STRANDEDNESS: single
TOPOLOGY: linear
MOLECULE TYPE: synthetic DNA
SEQUENCE DESCRIPTION: SEQ ID NO: 44
CACTGTCCCT ACCCGGGCAG CC 22

SEQ ID NO: 45
SEQUENCE LENGTH: 23
SEQUENCE TYPE: nucleic acid
STRANDEDNESS: single
TOPOLOGY: linear
MOLECULE TYPE: synthetic DNA
SEQUENCE DESCRIPTION: SEQ ID NO: 45
ATCTACCCAA GAAGGCTCGG CAC 23

SEQ ID NO: 46
SEQUENCE LENGTH: 23
SEQUENCE TYPE: nucleic acid
STRANDEDNESS: single

TOPOLOGY: linear
MOLECULE TYPE: synthetic DNA
SEQUENCE DESCRIPTION: SEQ ID NO: 46
TCTGTTAGGA GGCGCTACCA TGC 23

SEQ ID NO: 47
SEQUENCE LENGTH: 35
SEQUENCE TYPE: nucleic acid
STRANDEDNESS: single
TOPOLOGY: linear
MOLECULE TYPE: synthetic DNA
SEQUENCE DESCRIPTION: SEQ ID NO: 47
AATTCGGTAC CAGCTGAAGC TACCCCACCA TGCCC 35

SEQ ID NO: 48
SEQUENCE LENGTH: 34
SEQUENCE TYPE: nucleic acid
STRANDEDNESS: single
TOPOLOGY: linear
MOLECULE TYPE: synthetic DNA
SEQUENCE DESCRIPTION: SEQ ID NO: 48
AGTCGCTAGC AGAGGAGAAC AGGCACCTCA AAAC 34

SEQ ID NO: 49
SEQUENCE LENGTH: 24
SEQUENCE TYPE: nucleic acid
STRANDEDNESS: single
TOPOLOGY: linear
MOLECULE TYPE: synthetic DNA
SEQUENCE DESCRIPTION: SEQ ID NO: 49
TGTTATTAAT GATCTGGAAG CACC 24

## Claims

1. A protein comprising any one of the amino acid sequences set forth in SEQ ID NOs: 4, 6, 9, or 38, or a protein comprising any one of said amino acid sequences having substitution, deletion, or addition of one or more amino acids and having cancer metastasis potency.

2. A protein encoded by DNA which hybridizes with DNA comprising any one of the nucleotide sequences set forth in SEQ ID NOs: 3, 5, 7, 8, or 37, said protein having cancer metastasis potency.

3. A DNA encoding the protein according to claim 1.

4. The DNA according to claim 3, comprising any one of the nucleotide sequences set forth in SEQ ID NOs: 3, 5, 7, 8, or 37.

5. A DNA hybridizing with a DNA comprising any one of the nucleotide sequences set forth in SEQ ID NOs: 3, 5, 7, 8, or 37, encoding a protein having cancer metastasis potency.

6. A vector carrying the DNA according to any one of claims 3 to 5.

7. A transformant harboring the vector according to claim 6.

8. A method for preparing the protein according to claim 1 or 2, comprising culturing the transformant set forth in claim 7.

9. A DNA specifically hybridizing with the DNA according to any one of claims 3 to 5, comprising at least 15 nucleotide residues.

10. An antisense DNA against the DNA according to any one of claims 3 to 5 or a portion thereof.

11. An antibody binding to the protein according to claim 1 or 2.

12. A method for screening a compound having cancer metastasis inhibitory ability, comprising steps of:
(a) contacting a test sample with the protein according to claim 1 or 2, and
(b) selecting compounds having the activity to bind to the protein according to claim 1 or 2.

13. A method for screening compounds having cancer metastasis inhibitory ability, comprising steps of:
(a) contacting test samples with cells expressing the protein according to claim 1 or 2,
(b) detecting the expression level of the protein according to claim 1 or 2 in cells contacted with test samples using the antibody set forth in claim 11, and
(c) selecting a compound which reduces the expression level of the protein according to claim 1 or 2 as compared with that in cells not contacted with test samples.
